(19) Europäisches Patentamt
European Patent Office
Office européen des brevets

(11) **EP 4 632 356 A1**

(12) **EUROPEAN PATENT APPLICATION**

(43) Date of publication:
**15.10.2025 Bulletin 2025/42**

(21) Application number: **24305579.5**

(22) Date of filing: **10.04.2024**

(51) International Patent Classification (IPC):
***G01N 21/55*** *(2014.01)* ***G01N 21/86*** *(2006.01)*
***G01N 21/33*** *(2006.01)* ***G01N 21/3563*** *(2014.01)*

(52) Cooperative Patent Classification (CPC):
**G01N 21/55; G01N 21/274; G01N 21/31; G01N 21/86; G01N 33/442;** G01N 21/33; G01N 21/3563; G01N 2021/558; G01N 2021/869

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
- **TotalEnergies OneTech**
  **92400 Courbevoie (FR)**
- **Fraunhofer-Gesellschaft zur Förderung der angewandten Forschung e.V.**
  **80686 München (DE)**

(72) Inventors:
- **HEIDRICH, Robert**
  **80686 MÜNCHEN (DE)**
- **MORDVINKIN, Anton**
  **80686 MUNCHEN (DE)**
- **GOTTSCHALG, Ralph**
  **80686 MUNCHEN (DE)**
- **BAINIER, Camille**
  **92400 COURBEVOIE (FR)**

(74) Representative: **Lavoix**
**2, place d'Estienne d'Orves**
**75441 Paris Cedex 09 (FR)**

# (54) METHOD FOR NON-DESTRUCTIVELY ASSESSING A LOCAL CONCENTRATION OF AT LEAST ONE ADDITIVE IN A POLYMER FILM

(57) A method (100, 6100) for non-destructively assessing a local concentration of at least one additive in a polymer film, the method comprising: a) providing a polymer film (15), comprising a polymer matrix and at least one additive dispersed in the matrix; b) irradiating a predetermined area (40) of the polymer film (15) positioned in an irradiation zone (35) with a light source (25) emitting a light comprising at least one irradiation wavelength (λ characteristic of at least one of the additives; c) measuring at least one of a transmittance (T(λ)) and a reflectance (R(A)) of the predetermined area (40) at the irradiation wavelength (λ); and d) determining a concentration of the at least one additive in the predetermined area (40), using the measured transmittance (T(λ)) and/or the measured reflectance (R(A)), and using at least one calibration relationship characteristic of the at least one additive and of the polymer.

FIG.2

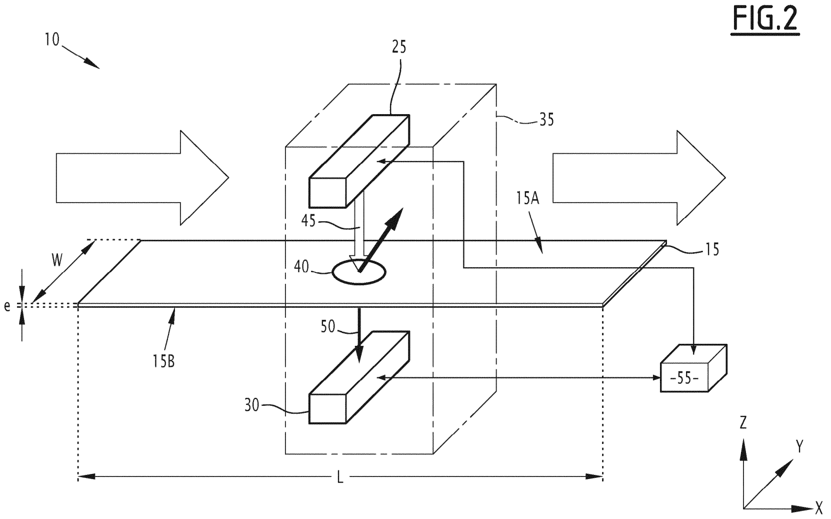




Processed by Luminess, 75001 PARIS (FR)

## Description

The present invention concerns a method and a device for non-destructively assessing a local concentration of at least one additive in a polymer film, such as a polymer film for encapsulating an electronic device, for example at least one photovoltaic cell.

Encapsulants are critical components in electronic devices such as photovoltaic modules or batteries. In addition to protecting photovoltaic cells from UV light, encapsulants may perform several functions. Among others, encapsulants provide optical coupling, mechanical stability, and support to other components of the photovoltaic modules, as well as protection of electrical components from environmental factors. They also provide electrical insulation for safety reasons.

Encapsulants can be supplied in the form of polymer films. However, degradation of the polymer films under the influence of temperature, humidity and/or irradiation might lead to degradation modes of the photovoltaic cells, such as delamination, corrosion, and discoloration.

In order to enhance the performances and/or the reliability of the encapsulating polymer films, various additives can be incorporated in the host polymer matrix, such as crosslinking additives, UV absorbers, UV stabilizers and/or antioxidants.

In general, to form polymer films incorporating specific additive(s), premixed pellets of a host polymer and of one or more appropriate additive masterbatches are fed in an extrusion machine, which extrudes the polymer film.

However, a homogeneous distribution of the additives in the host polymer matrix is sometimes quite difficult to achieve. Therefore, at some regions of the polymer film, local concentration of one or more additives may not meet the required specifications.

Assessing the local concentration of an additive in a polymer film is thus essential to qualify the polymer film for subsequent use, for example in an electronic device such as a photovoltaic cell or in a packaging, or to qualify a device comprising an encapsulant in the form of a polymer film, such as an electronic device, for example a photovoltaic cell, a chip or a battery.

The article "Quantification of UV protecting additives in ethylene-vinyl acetate copolymer encapsulants for photovoltaic modules with pyrolysis-gas chromatography-mass spectrometry", Heidrich R. et al., Polymer Testing, 2023, vol. 118, p. 107913, describes a method for quantifying UV additives in ethylene vinyl acetate copolymer (EVA) films. The method comprises preparing calibration samples of polymer film having a known concentration of chosen additives; determining an offset corrected calibration function; and performing a quantitative Pyrolysis - Gas Chromatography - Mass Spectrometry (PY-GCMS) analysis of an unknown sample of a polymer film to be assessed based on the offset corrected calibration function.

However, this quantitative method is destructive, cumbersome and allows assessment of only small sample areas.

Hence, one aim of the invention is to provide a method for assessing a local concentration of at least one additive in a polymer film for encapsulating at least one photovoltaic cell which is non-destructive, easy to implement, and allows assessing a larger area of the polymer film.

To this aim, the subject-matter of the invention is a method for non-destructively assessing a local concentration of at least one additive in a polymer film, the method comprising:

a) providing a polymer film, comprising a polymer matrix and at least one additive dispersed in the matrix;
b) irradiating a predetermined area of the polymer film positioned in an irradiation zone with a light source emitting a light comprising at least one irradiation wavelength, the irradiation wavelength being characteristic of at least one of the additives;
c) measuring at least one of a transmittance and a reflectance of the predetermined area at the at least one irradiation wavelength; and
d) determining a concentration of the at least one additive in the predetermined area of the polymer film, using the measured transmittance and/or the measured reflectance, and using at least one calibration relationship, the calibration relationship being characteristic of the at least one additive and of the polymer.

Unexpectedly in the field of polymer films, the inventors have observed that it is possible to determine a calibration relationship, which is characteristic of the at least one additive and of the polymer and relates either the transmittance or the reflectance and the concentration of the at least one additive.

This calibration relationship makes it possible to non-destructively assess a local concentration of the at least one additive, by analyzing the light either transmitted or reflected by the predetermined area in response to an irradiation of a predetermined area of the polymer film.

Hence, the method according to the invention is not destructive, easy to implement and allows a fast assessment of a local concentration of the at least one additive in the polymer film.

The simplicity of the method and its short response time allow fast analysis of large lengths of a polymer film, the analysis being if needed carried out over an entire width of the polymer film.

The method according to the invention may comprise one or more of the following feature(s), taken solely, or according to any technical feasible combination:

- the method comprises continuously relatively moving the polymer film and the irradiation zone along a moving direction and determining a concentration of the at least one additive at a plurality of positions in the polymer film along the moving direction;
- the method comprises an initial wavelength determination step comprising: obtaining a reflectance spectrum and/or a transmittance spectrum of a calibration sample as a function of wavelength, the calibration sample having a known concentration of the at least one additive in the polymer matrix, and determining at least one irradiation wavelength using the reflectance spectrum and/or the transmittance spectrum;
- the at least one irradiation wavelength corresponds to an absolute or local maximum of absorption of the transmittance spectrum or/and of the reflectance spectrum or to an absolute or local minimum of the transmittance spectrum and/or of the reflectance spectrum or to an inflection point of the reflectance spectrum;
- the method comprises an initial calibration step, the initial calibration step comprising:

  i) providing a set of calibration samples of polymer films, each calibration sample having a known concentration of the at least one additive in the polymer matrix;
  ii) irradiating a predetermined area of each calibration sample in the irradiation zone with the light source;
  iii) measuring at least one of a transmittance and a reflectance of said predetermined area; and
  iv) determining the at least one calibration relationship based on said measurements;

- at step i), a set of multilayered calibration coupons are provided, at least one layer of each coupon being formed with a respective calibration sample, the irradiation being performed at step ii) on said calibration coupons;
- the polymer is chosen among a polypropylene (PP), a polyethylene terephthalate (PET), an ethylene vinyl acetate copolymer (EVA), a polyolefin elastomer (POE) and a thermoplastic polyolefin (TPO);
- the additive is chosen among UV absorbers, UV stabilizers, crosslinking additives, and/or antioxidants;
- the polymer film is intended for encapsulating an electronic device chosen among a photovoltaic cell, a battery, a chip;
- the polymer film encapsulates at least one photovoltaic cell of a photovoltaic panel, the light source irradiating an upper surface of the photovoltaic panel opposite the polymer film relative to the photovoltaic cell, a reflectance being measured;
- the method comprises measuring the local concentration of the at least one additive in the polymer film alone in a preliminary assessment step before its attachment in the photovoltaic panel, a transmittance being measured for the preliminary assessment;
- if a transmittance is measured, the calibration relationship comprises the following equation: $C = -\frac{\ln(transmittance)}{SF}$, where SF is a scaling factor characteristic of the at least one additive and of the polymer and C is the concentration of the at least one additive in the polymer matrix;
- the calibration relationship is determined from a calibration curve relating a known concentration of the additive in a calibration polymer matrix identical to the polymer matrix and a reflectance.

Another subject matter of the invention is a device for non-destructively assessing a local concentration of at least one additive in a polymer film intended for or encapsulating at least one photovoltaic cell comprising:

- at least one light source, configured to emit a light comprising at least one irradiation wavelength in an irradiation zone, the irradiation wavelength being characteristic of at least one of the additives;
- at least one detector, configured to detect a light transmitted and/or reflected by a predetermined area of a polymer film positioned in the irradiation zone and for determining a reflectance and/or a transmittance based on the detected light and the irradiation light;
- a processor, configured to determine a concentration of the at least one additive in the predetermined area of the polymer film, using the measured transmittance and/or the measured reflectance and on at least one calibration relationship, the calibration relationship being characteristic of the at least one additive and of the polymer matrix.

The device according to the invention may comprise a moving system configured to continuously relatively move the polymer film and the irradiation zone along at least one moving direction.

The invention will be better understood based on the following description, given solely as an example, and made in reference to the appended drawings, in which:

- Figure 1 shows an exemplary multilayered photovoltaic panel;
- Figure 2 is a schematic representation of a first embodiment of the device according to the invention;
- Figure 3 is a graph representing the transmittance spectrum of each sample of the set of calibration samples of example 1 as a function of the irradiation wavelength;
- Figure 4 is a graph representing the transmittance of each of the calibration samples of example 1 measured at a wavelength of 265.0 nm as a function of the known mass fraction of an example of UV absorber, namely Cyasorb® UV531;
- Figure 5 shows the mass fraction of Cyasorb® UV531 measured for each sample of the set of validation samples of example 1 as a function of the expected mass fraction of Cyasorb® UV531;
- Figure 6 is a schematic representation of a second embodiment of the device according to the invention;
- Figure 7 is a graph representing the reflectance spectra of coupons formed with each sample of the set of calibration samples of example 1, as a function of the irradiation wavelength;
- Figure 8 is a graph representing the reflectance of a coupon formed with the first calibration sample of example 1 and the derivative of this reflectance as a function of the irradiation wavelength;
- Figure 9 is a graph representing the reflectance of coupons formed with each of the calibration samples of example 1 measured at a wavelength of 381 nm, which is the wavelength of the inflection point of figure 6, as a function of the known mass fraction of Cyasorb® UV531;
- Figure 10 shows the mass fraction of Cyasorb® UV531 measured for each sample of the set of validation samples of example 1, as a function of the expected mass fraction of Cyasorb® UV531;
- Figure 11 shows a flow chart of a first exemplary embodiment of the method according to the invention;
- Figure 12 shows a flow chart of a second exemplary embodiment of the method according to the invention;
- Figure 13 is a graph representing the transmittance spectrum of each sample of the set of samples of example 2 as a function of the irradiation wavelength;
- Figure 14 is a graph representing the transmittance of each sample of example 2 measured at a wavelength of 373.0 nm as a function of the known mass fraction of an example of UV absorber, namely Tinuvin® 329;
- Figure 15 is a graph representing the reflectance spectrum of coupons formed with each sample of example 2, as a function of the irradiation wavelength;
- Figure 16 is a graph representing the reflectance of a coupon formed with the first sample of example 2 and the derivative of this reflectance as a function of the irradiation wavelength;
- Figure 17 is a graph representing the reflectance of coupons formed with each of the three calibration samples of example 2 measured at a wavelength of 393 nm, which is the wavelength of the inflection point of figure 16, as a function of the known mass fraction of Tinuvin® 329; and
- Figure 18 shows the mass fraction of Tinuvin® 329 measured for coupons formed with each of the set of the three validation samples of example 2, as a function of the expected mass fraction of Tinuvin® 329.

In a first embodiment represented in figure 2, the device 10 for non-destructively assessing a local concentration of at least one additive in a polymer film 15, for example a polymer film 15 intended for or encapsulating at least one photovoltaic cell 20, comprises a light source 25 and a light detector 30 in an irradiation zone 35, and a moving system (not represented) configured to move the polymer film 15 with respect to the irradiation zone 35 along a moving direction X.

The polymer film 15 of figure 2 is intended for encapsulating the at least one photovoltaic cell 20.

To this end, the polymer film 15 comprises a polymer matrix.

The polymer matrix may comprise a polymer chosen among an ethylene vinyl acetate copolymer (EVA), a polyolefin, such as a polypropylene (PP) or a polyethylene (PE) or mixtures thereof, in particular a polyolefin elastomer (POE) or a thermoplastic polyolefin (TPO), polyethylene terephthalate (PET), polyvinyl butyral (PVB), and/or polydimethylsiloxane (PDMS).

The polymer film 15 comprises one or more additives dispersed in the polymer matrix, in order to enhance or modulate the performances and/or the reliability of the polymer film 15.

The performances of the polymer film 15 can include among others one or more optical, thermal or chemical property.

The reliability of the polymer film 15 can include among others a resistance to ageing. The additive is for example chosen among crosslinking additives, UV absorbers, UV stabilizers and/or antioxidants.

A crosslinking additive is configured to enhance the thermal stability and/or the mechanical properties of the polymer film 15 by forming covalent and/or ionic bonds between a plurality of polymer chains. Examples of crosslinking additives are crosslinking peroxides, such as Luperox® TBEC ((*tert*-Butylperoxy 2-ethylhexyl carbonate), and crosslinking accelerators, such as Triallylisocyanurate (TAIC).

A UV absorber is a compound able to absorb UV radiations and convert these radiations into heat, which is subsequently dissipated throughout the polymer film 15. Examples of UV absorbers are Cyasorb® UV531 (2-hydroxy-4-n-Octoxy Benzophenone), Cyasorb® UV9 (2-hydroxy-4-methoxy-benzophenone), Tinuvin® 328 (2-(2H-Benzotriazol-2-yl)-4,6-ditertpentylphenol), or Tinuvin® 329 (2-(2H-Benzotriazol-2-yl)-4-(1,1,3,3-tetramethylbutyl)phenol).

A UV stabilizer is a compound configured to inhibit or delay the UV-induced degradation of the polymer film 15. For example, a UV stabilizer may competitively absorb UV radiations before they can reach chromophore(s) in the polymer matrix, or it may deactivate or quench excited species generated by the UV radiation, or it may scavenge reactive free radicals generated by UV radiations. Examples of UV stabilizers are Tinuvin® 770 (Bis(2,2,6,6-tetramethyl-4-piperidyl) sebacate), Tinuvin® 123 (Decanedioic acid, bis (2,2,6,6-tetramethyl-1-(octyloxy)-4-piperidinyl) ester) or Tinuvin® 292 (Bis(1,2,2,6,6-pentamethyl-4-piperidyl) sebacate).

Examples of anti-oxidants are butylated hydroxytoluene (BHT, 2,6-di-tert-butyl-4-methylphenol), Naugard® P (Tris(nonylphenyl) phosphite), Naugard ® 524 (Tris(2,4-di-tert-butylphenyl) phosphite) or D 16-834 (Tris(2,4-di-tert-butylphenyl)phosphate).

The polymer film 15 extends along a longitudinal direction, which is the same as the moving direction X in the case of figure 2, and along a transverse direction Y.

The polymer film 15 also extends along an elevation direction Z between an upper face 15A and a lower face 15B.

A thickness e of the polymer film 15 along the elevation direction Z is substantially less that a length L and a width W of the polymer film 15 respectively along the longitudinal direction X and the transverse direction Y.

Typically, the thickness e is of the order of 500 $\mu$m or less.

The moving system is configured to move the polymer film 15 with respect to an irradiation zone 35 at least along the moving direction X.

The moving system is preferably configured to continuously move the polymer film along the moving direction X.

For example, the moving system comprises a conveyor having a drive roller and means configured to tension the polymer film 15 at least in the irradiation zone 35.

The light source 25 is positioned in the irradiation zone 35. It is configured to emit a light comprising at least one irradiation wavelength $\lambda$ towards a predetermined area 40 of the upper face 15A of the polymer film 15 comprised in the irradiation zone 35.

In the example of figure 1, the light source 25 is configured to emit a single substantially cylindrical irradiation beam 45. In this case, the predetermined area 40 consists of one single disk.

Alternatively, the light source 25 is configured to emit several overlapping or advantageously non-interfering irradiation beams 45, which are distributed along the transverse direction Y.

The at least one irradiation wavelength $\lambda$ is advantageously chosen according to the additive and/or the polymer matrix.

The at least one irradiation wavelength $\lambda$ is for example comprised in the UV range. Alternatively, the at least one irradiation wavelength $\lambda$ is comprised in the visible range or in the IR range.

Advantageously, the light source 25 is tunable.

The light source 25 comprises a controller configured to control an intensity $I_0(\lambda)$ of the light emitted at the respective irradiation wavelength $\lambda$.

The light detector 30 is positioned in the irradiation zone 35 so as to detect one transmitted beam 50, or a plurality of transmitted beams 50 if applying, the transmitted beams 50 being transmitted by the predetermined area 40 in response to the irradiation with the light source 25.

Typically, the light detector 30 is positioned opposite the light source 25 with respect to the polymer film 15 along the elevation direction Z.

Advantageously, the light detector 30 comprises a filter configured to filter the at least one irradiation wavelength $\lambda$.

The device 10 further comprises a processor 55 configured to exchange data with the light source 25 and the light detector 30.

The processor 55 is configured to determine a concentration of the at least one additive in the predetermined area 40, using data received from the light source 25 and from the light detector 30 and using at least one calibration relationship relating a measured transmittance and a concentration of the at least one additive in the polymer matrix. The calibration relationship is thus characteristic of the at least one additive and of the polymer.

A method 100 for non-destructively assessing a local concentration of at least one additive in the polymer film 15 according to the invention and carried out with the device 10 of figure 2 is described hereafter with reference to figure 11.

The method 100 comprises the provision 110 of the polymer film 15, the irradiation 120 of the predetermined area 40 of the polymer film 15, the measurement 130 of a transmittance of the predetermined area 40 and the determination 140 of a concentration of the at least one additive in the predetermined area 40, using the measured transmittance and the at least one calibration relationship.

In the provision step 110, the polymer film 15 is positioned on or in the moving system.

The polymer film 15 is provided either right after exiting a production line configured to produce the polymer film 15 or after a storage delay after its production, for example shortly before the attachment of the polymer film 15 in the photovoltaic film in an assembling line.

Advantageously, the moving system is coupled with a polymer film production line and/or with an assembling line, such that the method 100 is continuously performed at an exit of the production line and/or before an entrance of the assembling line.

In the provision step 110, the moving system displaces the polymer film 15 relatively to the irradiation zone 35 along the moving direction X, and if required along the transverse direction Y, until the predetermined area 40 of interest of the polymer film 15 to be analyzed is in the irradiation zone 35.

Then, in the irradiation step 120, the predetermined area 40 is then irradiated with the light source 25 in the irradiation zone 35.

Part of the irradiation light is transmitted by the predetermined area 40 of the polymer film 15, such that the transmitted beam 50 is formed.

At the measurement step 130, the light detector 30 detects and analyzes the transmitted beam 50.

The transmitted beam 50 spectrum comprises the at least one irradiation wavelength $\lambda$.

The transmittance $T(\lambda)$ of the predetermined area 40 at the at least one irradiation wavelength is then determined with the processor 55, the transmittance $T(\lambda)$ being the ratio of the intensity $I(\lambda)$ of the transmitted light detected at the at least one irradiation wavelength $\lambda$ and of the intensity $I_0(\lambda)$ of the irradiating light.

Advantageously, several transmittances are determined at the measurement step 130, each corresponding to a respective irradiation wavelength $\lambda$.

This provision is advantageous for example in case the polymer film 15 comprises several additives, such as several UV absorbers. In this case, the irradiation can be performed with a plurality of irradiation wavelengths $\lambda$, for example in quick succession, and the transmittance $T(\lambda)$ of the predetermined area 40 is determined for each of the irradiation wavelengths $\lambda$.

Last, the processor 55 determines at the determination step 140 the concentration of the at least one additive in the predetermined area 40, using the at least one calibration relationship and the transmittance $T(\lambda)$.

The calibration relationship is advantageously determined in an initial calibration step 150.

The initial calibration step 150 comprises an initial provision step 151, at which of a set of N calibration samples of polymer films is provided, N being a positive integer.

Each calibration sample i (i being an integer comprised between 1 and N) comprises/

- a polymer matrix having the same composition as the polymer matrix of the polymer film 15 to be analyzed, and
- the at least one additive of interest dispersed in the polymer matrix in a known concentration c_th_i, or equivalently in a known weight percentage WPth_i with respect to the total weight of the polymer film.

The initial calibration step 150 further comprises an initial irradiation step 152 at which a predetermined area of each calibration sample is provided in the irradiation zone 35 is irradiated with the light source 25 at the at least one irradiation wavelength $\lambda$.

The transmittance $T_i(\lambda)$ of each calibration sample is then determined at an initial measurement step 153 with the processor 55, based on the data transmitted by the light source 25 and the light detector 30 detecting the light transmitted by the predetermined area of the calibration sample.

The calibration relationship is then determined at an initial determination step 154 by a processor, based on a mathematical model which best fits the data set $\{WPth_i, T_i(\lambda)\}$ for i ranging from 1 to N.

Figure 4 illustrates the initial calibration step 150 in the case of the exemplary set of calibration samples of example 1. In this case, the determined calibration relationship is:
Equation 1

$$WP = -\frac{\ln T(\lambda)}{SF} \qquad (1)$$

where WP is the weight percentage of the at least one additive of interest and SF a scaling factor, which is characteristic of the polymer and the at least one additive.

In the case of figure 4, the scaling factor SF was found to be equal to 12.5 per weight % with the light source 25 and the light detector 30 used for example 1, this scaling factor being characteristic of EVA and of the UV absorber Cyasorb® UV 531. It is worth to note that Tinuvin® 770 is a light stabilizer, which acts as radical scavenger and does not absorb UV radiations.

The same type of relationship is observed in the case of example 2. Based on figure 14, the scaling factor SF was found to be equal to 15 per weight %.

The calibration relationship of equation 1 recalls the Beer-Lambert's law in a totally unexpected manner. In particular, the scattering effects in the polymer films 15 were expected to render the Beer-Lambert's law inapplicable, which explains that only implemented destructive assessment methods have been implemented to date.

Figure 3 and 13 confirm the unexpected nature of this observation, since it shows that the UV-absorber content has very diverse influences on the transmission behavior of the polymer film 15. Among others, the horizontal arrow on figure 3 shows that the 50% transmission cutoff shifts from 354 nm to 376 nm as the additive concentration increases, the shift being non linear with respect to the additive concentration. Moreover, local maxima can be observed or not depending on the additive concentration, a local maximum being observed at 265 nm for concentrations up to $2c_0$, while a local maximum is observed at 315 nm for concentrations only up to ¾ $c_0$.

As an alternative embodiment, the calibration relationship may be based on the shift of a wavelength of a n% transmission cutoff, n being for example equal to 50, relative to a wavelength of a n% transmission cutoff of the polymer film 15 without the additive of interest.

Without wishing to be bound by a theory, the inventors are of the opinion that the calibration relationship of the above equation 1 is applicable to a wide range of polymer films 15 provided an adaptation of the irradiation wavelength and of the scaling factor SF to the nature of the polymer matrix and of the additive, and optionally to a particular range of expected additive concentration.

Figure 5 was obtained by comparing the weight percentages WPmeas obtained with the method 100 for each of the five validation samples of example 1 to the expected weight percentages WPth.

Figure 5 shows that the calibration relationship of equation 1 makes it possible to non-destructively estimate the weight percentage of Cyasorb® UV 531 with a very good precision at least in the range [0%, 0.30%] in the case of example 1.

This range of weight percentages of UV absorber is particularly interesting since frequently implemented in commercially available polymer films, as described for example in "Quantification of UV protecting additives in ethylene-vinyl acetate copolymer encapsulants for photovoltaic modules with pyrolysis-gas chromatography-mass spectrometry", Heidrich R. et al., Polymer Testing, 2023, vol. 118, p. 107913.

Advantageously, the method 100 comprises an initial wavelength determination step 160.

The initial wavelength determination step 160 comprises obtaining at least one transmittance spectrum of at least one calibration sample as a function of wavelength, the calibration sample having a known concentration of the at least one additive in the polymer matrix.

The calibration sample of the initial wavelength determination step 160 is advantageously one of the calibration samples used at the calibration step 150.

The transmittance spectrum of is obtained by irradiating a predetermined area of the calibration sample simultaneously or successively at a plurality of wavelengths with a light source.

The light source is advantageously the light source 25.

The light transmitted by the predetermined area is detected with a detector, advantageously the light detector 30, and a transmittance of the calibration sample for each of the plurality of wavelengths is determined with a processor, advantageously the processor 55.

Figure 3 shows the transmittance spectra obtained at the initial wavelength determination step 160 for each of the ten calibration samples of example 1 in the wavelength range [200 nm; 700 nm].

Figure 13 shows the transmittance spectra obtained at the initial wavelength determination step 160 for each of the six samples of example 2 in the wavelength range [200 nm; 700 nm].

The determination step 160 further comprises determining at least one irradiation wavelength using the at least one transmittance spectrum.

Advantageously, at least one irradiation wavelength $\lambda$ corresponds to a maximum of the at least one transmittance spectrum.

Figure 3 shows that in the case of example 1, a local maximum occurs in the transmittance spectra of the calibration samples of example 1 at an irradiation wavelength of 265 nm whatever the weight percentage of Cyasorb® UV 531. The transmittance at 265 nm interestingly shows a non-linear dependency on the Cyasorb® UV 531 weight percentage. Therefore, the irradiation wavelength of 265 nm was chosen for the initial calibration step 150 of figure 4.

This choice subsequently appeared to be appropriate but other wavelengths may be tried and tested at the initial calibration step 150, such as a wavelength of 315 nm, corresponding to another local maximum of the transmittance spectra of figure 3 for the lower concentrations of additive of example 1.

In the same manner, Figure 13 shows that in the case of example 2, a local maximum occurs in the transmittance spectra of the samples of example 2 at an irradiation wavelength of 265 nm whatever the weight percentage of Tinuvin® 329. The transmittance at 265 nm interestingly shows a non-linear dependency on the Tinuvin® 329 weight percentage, as well as the transmittance at 373 nm, not corresponding to a local maximum as can be observed on figure 14. In this case, the irradiation wavelength of 373 nm was chosen for the initial calibration step 150 based on the results of figure 14.

Advantageously, the moving system continuously moves the polymer film 15 along the moving direction X. The irradiation step 120, the measurement step 130 and the determination step 140 are repeated at successive measurement instants, e.g. periodically, such that the local concentration of the at least one additive is assessed repeatedly, e.g.

periodically, along the length L of the polymer film 15.

Advantageously, the light source irradiates the polymer film 15 simultaneously with a plurality of irradiation beams 45 distributed, preferably equally distributed, along the transverse direction Y, such that the local concentration of the at least one additive is assessed along the width W of the polymer film 15.

In a particular embodiment, the method 100 is carried out on a polymer film 15 encapsulating at least one photovoltaic cell 20.

The method 100 is easy to implement, in particular online a production line, fast and non-destructive.

The method 100 makes it possible to control the homogeneity of the concentration of additives in the polymer film 15 either alone or encapsulating at least one photovoltaic cell 20 by appropriately choosing the predetermined areas 40 successively and/or simultaneously irradiated.

A second embodiment of the device for non-destructively assessing a local concentration of at least one additive in a polymer film 15 intended for or encapsulating at least one photovoltaic cell 20 according to the invention is shown in figure 6.

The device 610 of this second embodiment differs from the device 10 of the first embodiment in that the light detector 630 is positioned in the irradiation zone 35 so as to receive one reflected beam 650, or a plurality of reflected beams 650 if applying, the reflected beams 650 being transmitted by the predetermined area 40 in response to the irradiation with the light source 25.

Typically, the light detector 630 is positioned on the same side as the light source 25 with respect to the polymer film 15 along the elevation direction Z.

In this case, the processor 655 is configured to determine a concentration of the at least one additive in the predetermined area 40, using data received from the light source 25 and from the light detector 630 and at least one calibration relationship relating a measured reflectance and a concentration of the at least one additive in the polymer matrix, the calibration relationship being thus characteristic of the at least one additive and of the polymer.

The method 6100 carried out with the device 610 is described in reference to figure 12.

The method 6100 differs from the method 100 in that, at the measurement step 6130, the light detector 630 detects and analyzes the reflected beam 650.

The reflectance $R(\lambda)$ of the predetermined area 40 at the at least one irradiation wavelength $\lambda$ is then determined with the processor 655, the reflectance $R(\lambda)$ being the ratio of the intensity $I(\lambda)$ of the light detected at the at least one irradiation wavelength $\lambda$ and of the intensity $I_0(\lambda)$ of the irradiating light.

Advantageously, several reflectances are determined, each corresponding to a respective irradiation wavelength $\lambda$.

This provision is advantageous for example in case the polymer film 15 comprises several additives, such as several UV absorbers. In this case, the irradiation can be performed with a plurality of irradiation wavelengths $\lambda$, for example in quick succession, and the transmittance $T(\lambda)$ of the predetermined area 40 is determined for each of the irradiation wavelengths $\lambda$.

Last, at the determination step 6140, the processor 655 determines the concentration of the at least one additive in the predetermined area 40, using the at least one calibration relationship and the reflectance $R(\lambda)$.

The calibration relationship is advantageously determined in an initial calibration step 6150.

The optional initial calibration step 6150 differs from the initial calibration step 150 in that the reflectance $R_i(\lambda)$ of each calibration sample is determined at an initial measurement step 6153 with the processor 655, based on the data transmitted by the light source 25 and the light detector 30 detecting the light reflected by the predetermined area of the calibration sample.

The initial calibration step 6150 further comprises an initial determination step 6154 differing form the initial determination step 154 in that the calibration relationship is determined by a processor, based on a mathematical model which best fits the data set {WPth_i, $R_i(\lambda)$} for i ranging from 1 to N.

Figure 9 illustrates the initial calibration step 6150 in the case of the exemplary set of calibration samples of example 1. In this case, it was surprisingly observed that the calibration relationship at a wavelength of 380 nm corresponding to an inflection point of the reflectance spectra of figure 7, was a linear relationship:

Equation 2

$$R(\lambda) = -20.46\,\mathrm{WP} + 25.04 \qquad (2)$$

where WP is the weight percentage of the at least one additive of interest.

Figure 10 was obtained by comparing the weight percentages WPmeas obtained with the method 6100 for each of the five validation samples of example 1 to the expected weight percentages WPth. Figure 10 shows that the calibration relationship of equation 1 makes it possible to non-destructively estimate the weight percentage of Cyasorb UV 531 with a very good precision at least in the range [0.20%, 0.50%] in the case of example 1.

Figure 17 illustrates the initial calibration step 6150 in the case of the exemplary set of calibration samples of example 2, namely samples 1, 3, and 5. In this case, it was again surprisingly observed that the calibration relationship at a wavelength of 393 nm, corresponding to an inflection point of the reflectance spectra of figure 15 - which can be observed more precisely in figure 16 - was a linear relationship:
Equation 3

$$R(\lambda) = -24.97\,\mathrm{WP} + 28.75 \qquad (3)$$

where WP is the weight percentage of the at least one additive of interest.
Figure 18 was obtained by comparing the weight percentages WPmeas obtained with the method 6100 for the validation samples 2, 4 and 6 of example 2 to the expected weight percentages WPth based on the linear relationship determined based on calibration samples 1, 3 and 5. Figure 18 shows that the calibration relationship of equation 3 makes it possible to non-destructively estimate the weight percentage of Tinuvin 329 with a very good precision at least in the range [0.14%, 0.46%] in the case of example 2.
Advantageously, the method 6100 comprises an initial wavelength determination step 6160.
The initial wavelength determination step 6160 differs from the initial wavelength determination step 160 in that it comprises obtaining at least one reflectance spectrum of at least one calibration sample as a function of wavelength, the calibration sample having a known concentration of the at least one additive in the polymer matrix.
Figure 7 shows the reflectance spectra obtained for each of the ten calibration samples of example 1 in the wavelength range [300 nm; 700 nm].
Figure 15 shows the reflectance spectra obtained for each of the six samples of example 2 in the wavelength range [300 nm; 700 nm].
The determination step 6160 further comprises determining at least one irradiation wavelength using the at least one reflectance spectrum.
Advantageously, at least one irradiation wavelength $\lambda$ corresponds to an inflection point of the reflectance spectrum.
Figures 7 and 8 show that in the case of example 1, an inflection point occurs in the reflectance spectrum at an irradiation wavelength of 380 nm whatever the weight percentage of Cyasorb® UV 531. The reflectance interestingly shows a higher dependency on the Cyasorb® UV 531 weight percentage at 380 nm than at other wavelengths in the wavelength range [300 nm; 700 nm], which allows to increase the resolution of the method 200.
Therefore, the irradiation wavelength of 380 nm was chosen for the initial calibration step 6150 of figure 9.
Figures 15 and 16 show that in the case of example 2, an inflection point again occurs in the reflectance spectrum, in this case at an irradiation wavelength of 393 nm, whatever the weight percentage of Tinuvin® 329. The reflectance interestingly shows a higher dependency on the Tinuvin® UV 329 weight percentage at 393 nm than at other wavelengths in the wavelength range [300 nm; 700 nm], which allows to increase the resolution of the method 200.
Therefore, the irradiation wavelength of 393 nm was chosen for the initial calibration step 6150 of figure 17.
Advantageously, the method 6100 is carried out on a polymer film 15 encapsulating at least one photovoltaic cell 20, such as the exemplary mutilayered photovoltaic panel 60 of figure 1.
The photovoltaic panel 60 comprises a front polymer film 15C and a back polymer film 150 encapsulating photovoltaic cells 20 interconnected by means of cell interconnectors 65.
The photovoltaic panel 60 also comprises an upper layer 70, for example an upper glass layer, and a lower layer 75, for example a lower backsheet or a lower glass layer.
The method 6100 allows to assess the front polymer film 15C in case the light source 25 irradiates the upper layer 70, and to assess the lower polymer film 150 in case the light source 25 irradiates the lower layer 75, provided the lower layer 70 or the upper layer 75 is at least partially transparent to the irradiating light.
The method 6100 is in particular carried out to assess the content at the boundaries between different photovoltaic cells 20, where no photovoltaic cell 20 is located below the front polymer film 15C.
Advantageously, the initial calibration step 6150 is performed on coupons corresponding to the product to be tested, i.e. the same layers except the polymer film 15 which has a different concentration of the additive of interest in each of the coupons. This provision has been implemented in the examples of figures 7 and 15.
In a third embodiment, the device 10 according to the first embodiment and the device 610 according to the second embodiment are combined in a single device according to the invention, comprising a first light detector 30 configured to detect the transmitted light and a second light detector 30 configured to detect the reflected light. In particular, the light source 25 may emit a light in a direction substantially normal to an upper surface of the polymer film 15. In this case, the first and second light detectors 30 may be configured to receive a light having a propagation direction substantially normal to the upper surface of the polymer film 15.
In this case, a concentration of the at least one additive in the predetermined area of the polymer film is determined using both the measured transmittance and the measured reflectance.

In a fourth embodiment, a device 10 according to the first embodiment and a device 610 according to the second embodiment are implemented at two different places in a production line comprising a first station configured to carry out polymer film production and a second station configured to attach the polymer film produced with at least one photovoltaic cell.

In this case, a concentration of the at least one additive in the predetermined area 40 of the polymer film 15 is determined at an exit of the first line part with the device 10 and a concentration of the at least one additive in the predetermined area 40 of the polymer film 15 is subsequently determined with the device 610.

**Example 1:**

Materials

A set of ten calibration samples and of five validation samples were prepared with the following chemicals:

- ELVAX® 150W (Ethylene Vinyl Acetate copolymer),provided by Safic Alcan Germany (DOW);
- Cyasorb® UV 531 (UV absorber), provided by Sigma Aldrich (2-hydroxy-4-n-Octoxy Benzophenone ; CAS number 1843-05-6);
- Tinuvin® 770 (hindered amine light stabilizer), provided by Sigma Aldrich (bis(2,2,6,6-tetramethyl-4-piperidyl)sebacate ; CAS number 52829-07-9);
- Luperox® TBEC (crosslinking additive), provided by Sigma Aldrich (tert-Butylperoxy 2-ethylhexyl carbonate ; CAS number 34443-12-4);
- Perkalink® 301 (crosslinking additive), provided by Sigma Aldrich (1,3,5-Triallyl-1 ,3,5-triazine-2,4,6(1H,3H,5H)-trione ; CAS number 1025-15-6);
- Silane A 174 (adhesion promoter), provided by Sigma Aldrich (3-(Trimethoxysilyl)propyl methacrylate ; CAS number 2530-85-0).

The weight percentages of the components for each of the ten calibration samples are indicated in Table 1 and 2 below, in which $c_0$ designates a reference concentration of the UV absorber Cyasorb® UV 531 implemented in calibration sample 4.

The reference concentration is advantageously close to the expected concentration in the polymer film 15 to be analyzed.

**Table 1: mass percentages of calibration samples n°1 to 5 of example 1**

| Calibration sample number | 1 | 2 | 3 | 4 | 5 |
|---|---|---|---|---|---|
| Cyasorb concentration | ¼ $c_0$ | ½ $c_0$ | ¾ $c_0$ | $c_0$ | 5/4 $c_0$ |
| ELVAX 150W (w %) | 94.927 | 94.843 | 94.743 | 94.651 | 94.590 |
| Cyasorb UV 531 (w%) | 0.073 | 0.142 | 0.216 | 0.284 | 0.354 |
| Tinuvin 770 (w%) | 0.032 | 0.061 | 0.093 | 0.125 | 0.157 |
| Luperox TBEC (w%) | 1.422 | 1.434 | 1.437 | 1.416 | 1.415 |
| Perkalink 301 (w%) | 2.405 | 2.360 | 2.367 | 2.371 | 2.345 |
| Silan A 174 (w%) | 1.143 | 1.161 | 1.145 | 1.153 | 1.139 |

**Table 2: mass percentages of calibration samples n°6 to 10 of example 1**

| Calibration sample number | 6 | 7 | 8 | 9 | 10 |
|---|---|---|---|---|---|
| Cyasorb concentration | 3/2 $c_0$ | 7/4 $c_0$ | 2 $c_0$ | 9/4 $c_0$ | 5/2 $c_0$ |
| ELVAX 150W (w %) | 94.489 | 94.383 | 94.267 | 94.168 | 94.077 |
| Cyasorb UV 531 (w%) | 0.425 | 0.493 | 0.565 | 0.635 | 0.705 |
| Tinuvin 770 (w%) | 0.184 | 0.217 | 0.249 | 0.279 | 0.305 |
| Luperox TBEC (w%) | 1.417 | 1.421 | 1.421 | 1.412 | 1.422 |
| Perkalink 301 (w%) | 2.343 | 2.350 | 2.360 | 2.363 | 2.342 |

(continued)

| Calibration sample number | 6 | 7 | 8 | 9 | 10 |
|---|---|---|---|---|---|
| Silan A 174 (w%) | 1.143 | 1.136 | 1.138 | 1.143 | 1.149 |

The weight percentages of the components for each of the five validation samples of example 1 are indicated in Table 3 below.

**Table 3: mass percentages of validation samples n°V1 to V5 of example 1**

| validation sample number | V1 | V2 | V3 | V4 | V5 |
|---|---|---|---|---|---|
| Cyasorb and Tinvuvin concentration | 1/3 $c_0$ | 2/3 $c_0$ | $c_0$ | 4/3 $c_0$ | 5/3 $c_0$ |
| ELVAX 150W (w %) | 94.872 | 94.776 | 94.652 | 94.552 | 94.415 |
| Cyasorb UV 531 (w%) | 0.097 | 0.193 | 0.288 | 0.382 | 0.474 |
| Tinuvin 770 (w%) | 0.044 | 0.083 | 0.123 | 0.161 | 0.204 |
| Luperox TBEC (w%) | 1.427 | 1.422 | 1.427 | 1.414 | 1.412 |
| Perkalink 301 (w%) | 2.415 | 2.356 | 2.362 | 2.358 | 2.354 |
| Silan A 174 (w%) | 1.144 | 1.170 | 1.149 | 1.133 | 1.142 |

Preparation of the EVA calibration and validation samples

The calibration samples and the validation samples were prepared with a kneader and a hydraulic press instead of the more common extrusion to ensure a highly homogeneous additive distribution inside the polymer matrix.

The chemicals were precisely weighed and placed into small sample containers before the kneader was filled in a two-step process:

- in a first step, approximately 25 g EVA were admixed with all the additives in the corresponding concentration, and
- in a second step, another approximately 25 g EVA were given to the same sample container to transfer additive residues from the sample container to the kneader, to prevent the cross-linking additives from sticking to the bottom and the edges of the sample containers.

The kneading was carried out in the Rheomix 3000 kneader with RheoDrive 7 as stirring unit at 70 °C with 50 rpm for 5 min.

The samples were then transferred to a Rucks KV 192.00 hydraulic press.

A metallic frame with 1 mm thickness in combination with a layer stack of 7×120 μm PTFE films ensured a polymer film thickness of approximately 160 μm after the pressing process performed in the frame at 50 bar and 70 °C for 6 min.

Preparation of coupons for reflection measurements

The encapsulants were manufactured to coupons comprising in this order: a glass layer, a first encapsulant layer, a second encapsulant layer and a backsheet.

The backsheet is the same for all coupons, such that the influence of the backsheet is the same for all samples. The backsheet of example 1 is a mono film comprising polyethylene terephthalate (PET) and a coating. The backsheet is fluorine-free.

The glass used for the glass layer is 3 mm thick, tempered and of a low-iron type. A transmission measurement of the plain glass can be found in Robert Heidrich, et al.. "UV lamp spectral effects on the aging behavior of encapsulants for photovoltaic modules", Solar Energy Materials and Solar Cells 266 (2024), p. 112674.

The lamination parameters were the same for all coupon compositions.

The lamination parameters are similar to the ones implemented for commercially available encapsulants having the same polymer and additive structure.

A Meier ICOLAM 10/08 laminator was used for lamination. The laminator was preheated to 55 °C.

Afterwards, all samples of a given encapsulant batch were placed inside the laminator. The laminator was evacuated for 6.5 min and heated to 80 °C.

Afterwards the coupons were pressed with 600 mbar and heated to 155 °C within 3 min. The temperature and pressure were hold for additional 15 min. Afterwards the laminator was cooled to 55 °C within 30 min still applying the 600 mbar pressure.

UV/VIS measurements

UV/VIS measurements were carried out in a Perkin Elmer Lambda 1050. As Light source a deuterium / wolfram lamp was used while the detector was a InGaAs / PbS semiconductor with photo multiplier. The device was used in transmission mode with integration sphere in a wavelength interval of 200 nm to 700 nm and in reflection mode with integration sphere in a wavelength interval of 300 nm to 700 nm. The monochromator increment was set to 1 nm.

**Example 2:**

Materials

A set of six calibration/validation samples were prepared with the following chemicals:

- ELVAX® 150W (Ethylene Vinyl Acetate copolymer),provided by Safic Alcan Germany (DOW);
- Tinuvin® 329, provided by Sigma Aldrich (2-(2H-Benzotriazol-2-yl)-4-(1,1,3,3-tetramethylbutyl)phenol, CAS number 3147-75-9);
- Tinuvin® 770 (hindered amine light stabilizer), provided by Sigma Aldrich (bis(2,2,6,6-tetramethyl-4-piperidyl)sebacate ; CAS number 52829-07-9);
- Luperox® TBEC (crosslinking additive), provided by Sigma Aldrich (tert-Butylperoxy 2-ethylhexyl carbonate ; CAS number 34443-12-4);
- Perkalink® 301 (crosslinking additive), provided by Sigma Aldrich (1,3,5-Triallyl-1,3,5-triazine-2,4,6(1H,3H,5H)-trione ; CAS number 1025-15-6);
- Silane A 174 (adhesion promoter), provided by Sigma Aldrich (3-(Trimethoxysilyl)propyl methacrylate ; CAS number 2530-85-0).

The weight percentages of the components for each of the six samples are indicated in Table 4 below, in which $c_0$ designates a reference concentration of the UV absorber Tinuvin® 329 implemented in sample 4.

The reference concentration is advantageously close to the expected concentration in the polymer film 15 to be analyzed.

**Table 4: mass percentages of calibration/validation samples n°1 to 6 of example 2**

| sample number | 1 calibration | 2 validation | 3 calibration | 4 validation | 5 calibration | 6 validation |
|---|---|---|---|---|---|---|
| Cyasorb concentrat ion | ¼ $c_0$ | ½ $c_0$ | ¾ $c_0$ | $c_0$ | 5/4 $c_0$ | 3/2 $c_0$ |
| ELVAX 150W (w %) | 98.990 | 98.912 | 98.793 | 98.711 | 98.596 | 98.503 |
| Tinuvin 329 (w%) | 0.083 | 0.158 | 0.233 | 0.294 | 0.379 | 0.451 |
| Tinuvin 770 (w%) | 0.030 | 0.0051 | 0.077 | 0.099 | 0.134 | 0.158 |
| Luperox TBEC (w%) | 0.303 | 0.293 | 0.300 | 0.304 | 0.296 | 0.293 |
| Perkalink 301 (w%) | 0.301 | 0.293 | 0.300 | 0.296 | 0.303 | 0.301 |
| Silan A 174 (w%) | 0.293 | 0.293 | 0.296 | 0.296 | 0.292 | 0.293 |

The preparation of the EVA calibration samples and the UV/VIS measurements were performed in the same manner as in example 1.

**Claims**

1. Method (100, 6100) for non-destructively assessing a local concentration of at least one additive in a polymer film, the method comprising:

   a) providing a polymer film (15), comprising a polymer matrix and at least one additive dispersed in the matrix;
   b) irradiating a predetermined area (40) of the polymer film (15) positioned in an irradiation zone (35) with a light source (25) emitting a light comprising at least one irradiation wavelength (λ), the irradiation wavelength (λ) being characteristic of at least one of the additives;
c) measuring at least one of a transmittance (T(λ)) and a reflectance (R(λ)) of the predetermined area (40) at the at least one irradiation wavelength (λ); and
d) determining a concentration of the at least one additive in the predetermined area (40) of the polymer film (15), using the measured transmittance (T(λ)) and/or the measured reflectance (R(λ)), and using at least one calibration relationship, the calibration relationship being characteristic of the at least one additive and of the polymer.

**2.** Method (100, 6100) according to claim 1, comprising continuously relatively moving the polymer film (15) and the irradiation zone (35) along a moving direction (X) and determining a concentration of the at least one additive at a plurality of positions in the polymer film (15) along the moving direction (X).

**3.** Method (100, 6100) according to any of the preceding claims, comprising an initial wavelength determination step (160, 6160) comprising:

- obtaining a reflectance spectrum and/or a transmittance spectrum of a calibration sample as a function of wavelength, the calibration sample having a known concentration of the at least one additive in the polymer matrix, and
- determining at least one irradiation wavelength using the reflectance spectrum and/or the transmittance spectrum.

**4.** Method (100, 6100) according to claim 3, in which the at least one irradiation wavelength corresponds to an absolute or local maximum of absorption of the transmittance spectrum or/and of the reflectance spectrum or to an absolute or local minimum of the transmittance spectrum and/or of the reflectance spectrum or to an inflection point of the reflectance spectrum.

**5.** Method (100, 6100) according to any one of the preceding claims, comprising an initial calibration step (150, 6150), the initial calibration step comprising:

i) providing a set of calibration samples of polymer films, each calibration sample having a known concentration of the at least one additive in the polymer matrix;
ii) irradiating a predetermined area of each calibration sample in the irradiation zone with the light source (25);
iii) measuring at least one of a transmittance and a reflectance of said predetermined area; and
iv) determining the at least one calibration relationship based on said measurements.

**6.** Method according to claim 5, in which at step i), a set of multilayered calibration coupons are provided, at least one layer of each coupon being formed with a respective calibration sample, the irradiation being performed at step ii) on said calibration coupons.

**7.** Method (100, 6100) according to any one of the preceding claims, in which the polymer is chosen among a polypropylene (PP), a polyethylene terephthalate (PET), an ethylene vinyl acetate copolymer (EVA), a polyolefin elastomer (POE) and a thermoplastic polyolefin (TPO).

**8.** Method (100, 6100) according to any one of the preceding claims, in which the additive is chosen among UV absorbers, UV stabilizers, crosslinking additives, and/or antioxidants.

**9.** Method (6100) according to any one of the preceding claims, in which the polymer film (15) is intended for encapsulating an electronic device chosen among a photovoltaic cell (20), a battery, a chip.

**10.** Method (6100) according to any one of the preceding claims, in which the polymer film (15) encapsulates at least one photovoltaic cell (20) of a photovoltaic panel (60), the light source (25) irradiating an upper surface of the photovoltaic panel opposite the polymer film (15) relative to the photovoltaic cell (20), a reflectance (R(λ)) being measured.

**11.** Method according to claim 10, comprising measuring the local concentration of the at least one additive in the polymer film (15) alone in a preliminary assessment step before its attachment in the photovoltaic panel, a transmittance (T(λ)) being measured for the preliminary assessment.

**12.** Method according to any one of the preceding claims, wherein, if a transmittance (T(λ)) is measured, the calibration relationship comprises the following equation: $C = -\frac{\ln(transmittance)}{SF}$, where SF is a scaling factor characteristic of the at least one additive and of the polymer and C is the concentration of the at least one additive in the polymer matrix.

**13.** Method (100, 6100) according to any one of the preceding claims, wherein the calibration relationship is determined from a calibration curve relating a known concentration of the additive in a calibration polymer matrix identical to the polymer matrix and a reflectance (R(λ)).

**14.** Device (10, 610) for non-destructively assessing a local concentration of at least one additive in a polymer film (15), the device comprising:

- at least one light source (25), configured to emit a light comprising at least one irradiation wavelength (λ) in an irradiation zone (35), the irradiation wavelength (λ) being characteristic of at least one of the additives;
- at least one detector (30,630), configured to detect a light transmitted and/or reflected by a predetermined area (40) of a polymer film (15) positioned in the irradiation zone (35) and for determining a reflectance (R(λ)) and/or a transmittance (T(λ)) based on the detected light and the irradiation light;
- a processor (55, 655), configured to determine a concentration of the at least one additive in the predetermined area (40) of the polymer film (15), using the measured transmittance and/or the measured reflectance and on at least one calibration relationship, the calibration relationship being characteristic of the at least one additive and of the polymer matrix.

**15.** Device (10, 610) according to claim 14, comprising a moving system configured to continuously relatively move the polymer film (15) and the irradiation zone (35) along at least one moving direction (X).

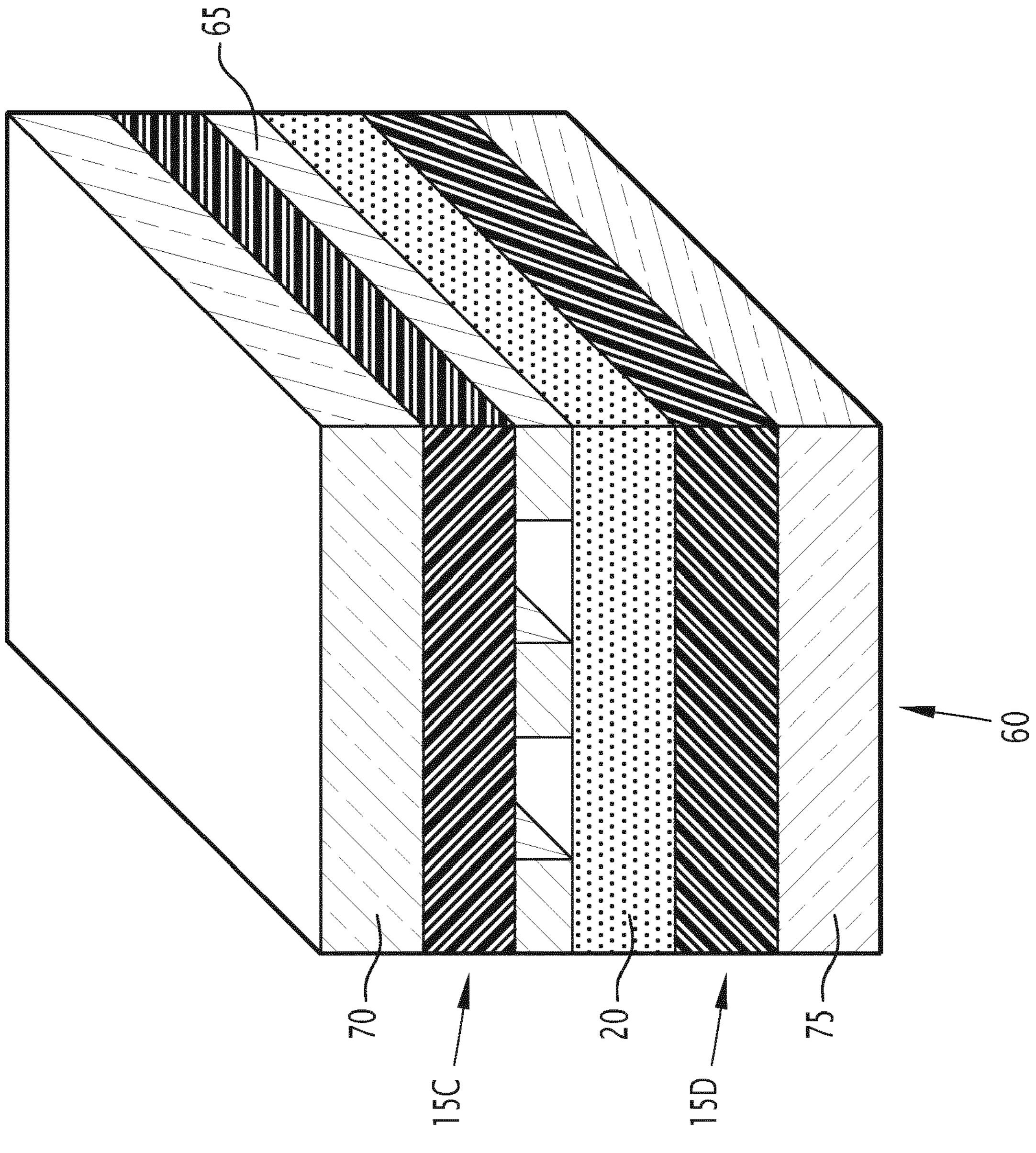


FIG.1

FIG.2

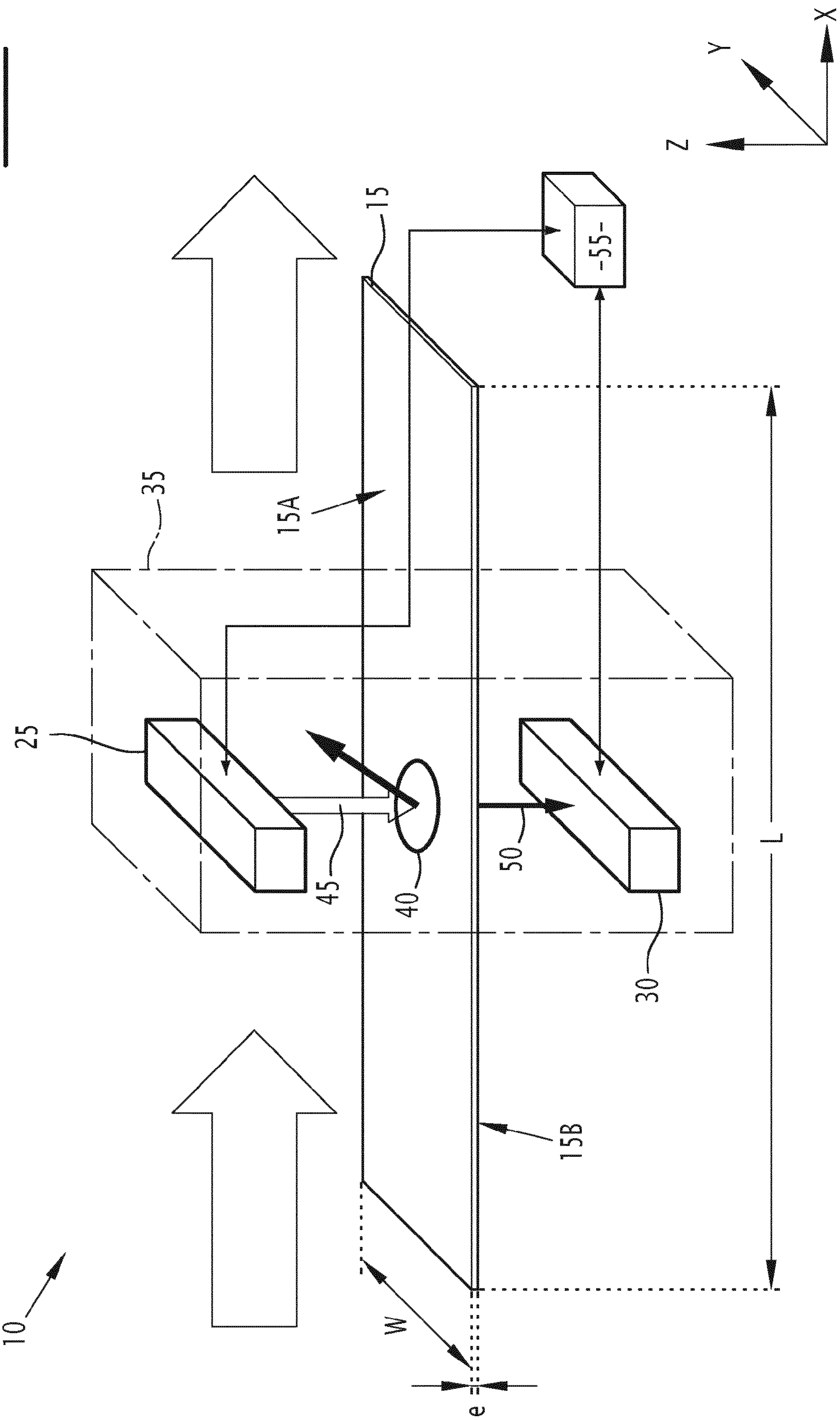

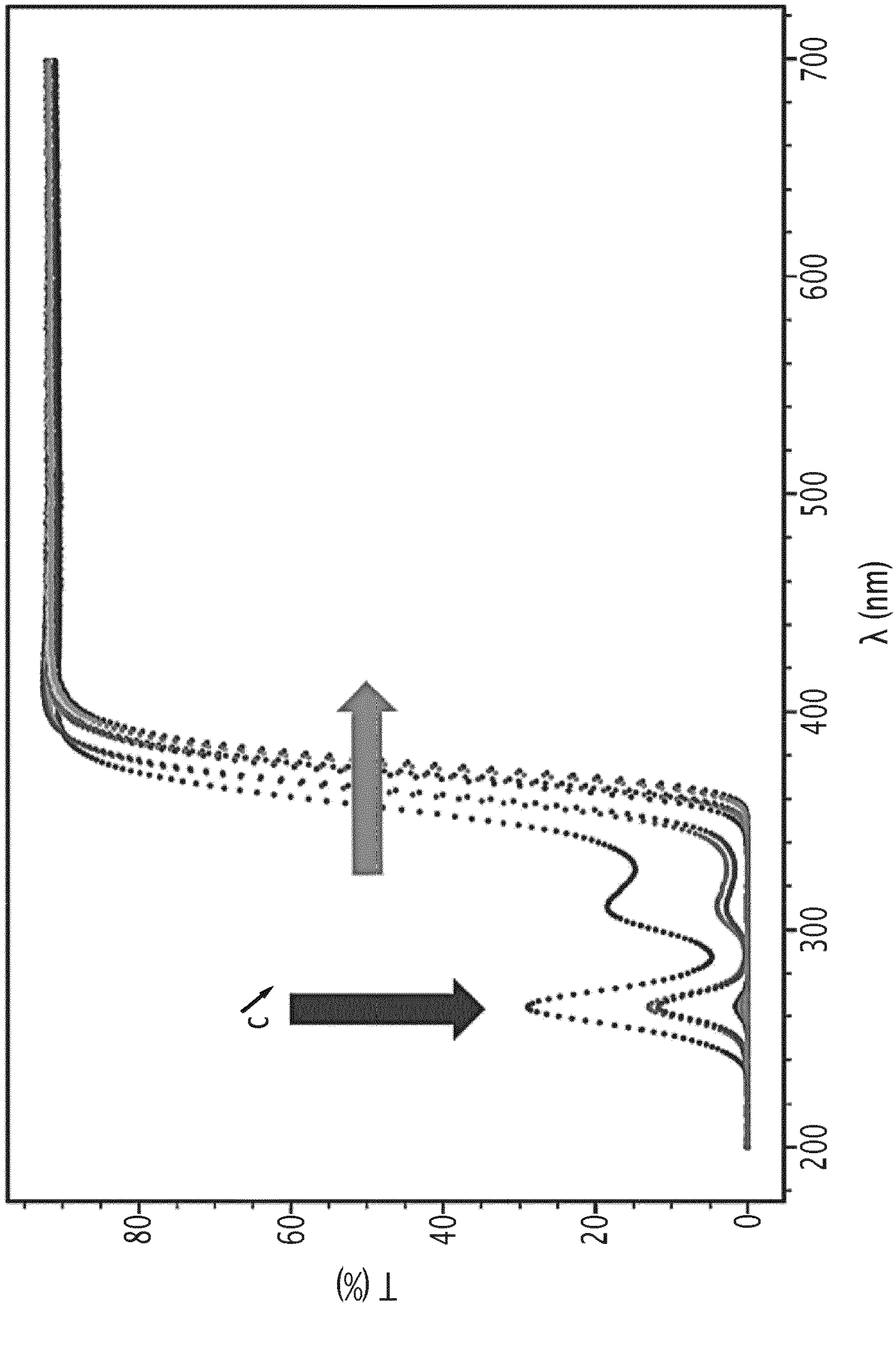


FIG.3

FIG.4

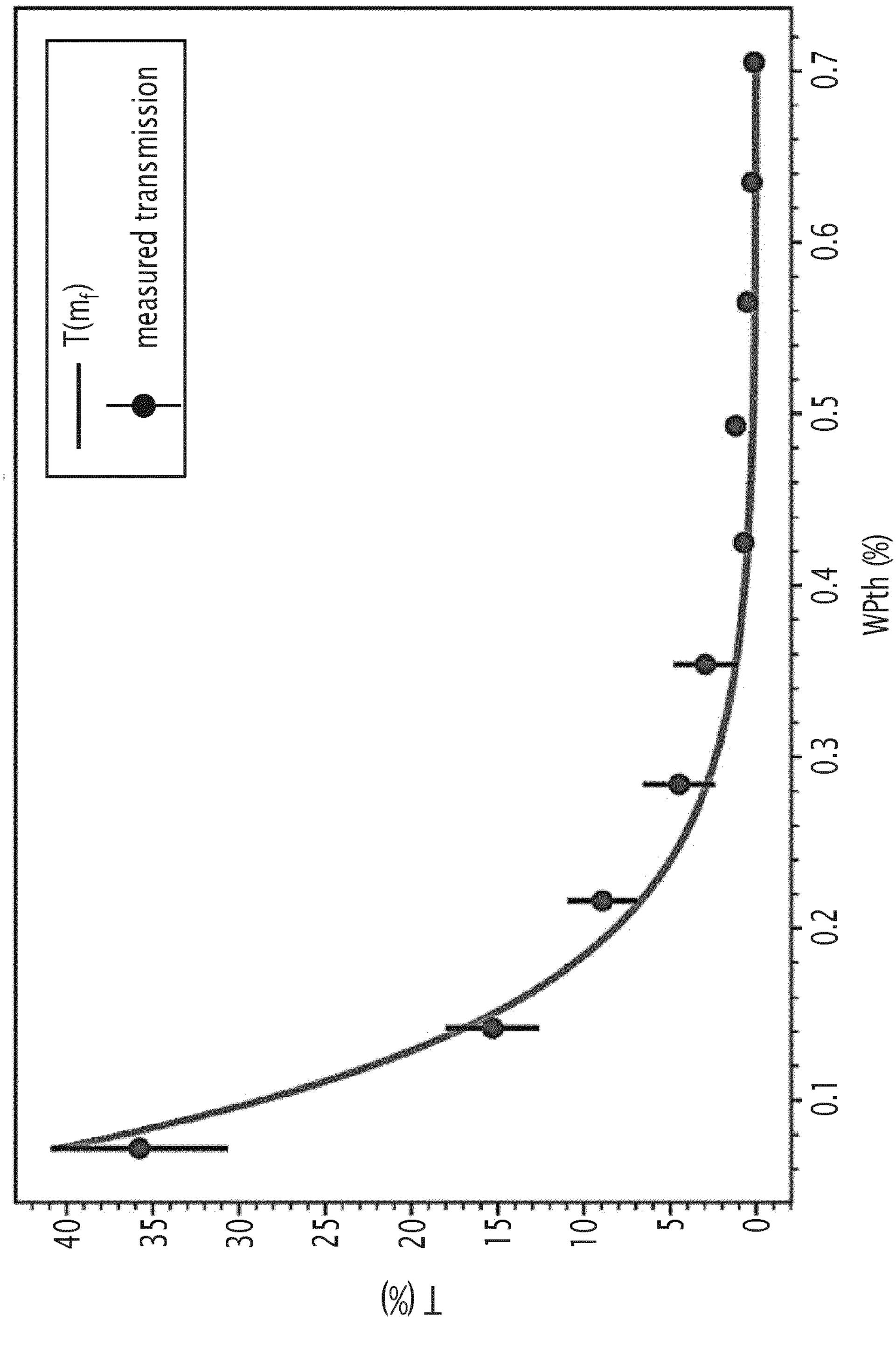
T(m$_f$)
measured transmission
T (%)
WPth (%)
0
5
10
15
20
25
30
35
40
0.1
0.2
0.3
0.4
0.5
0.6
0.7

FIG.5

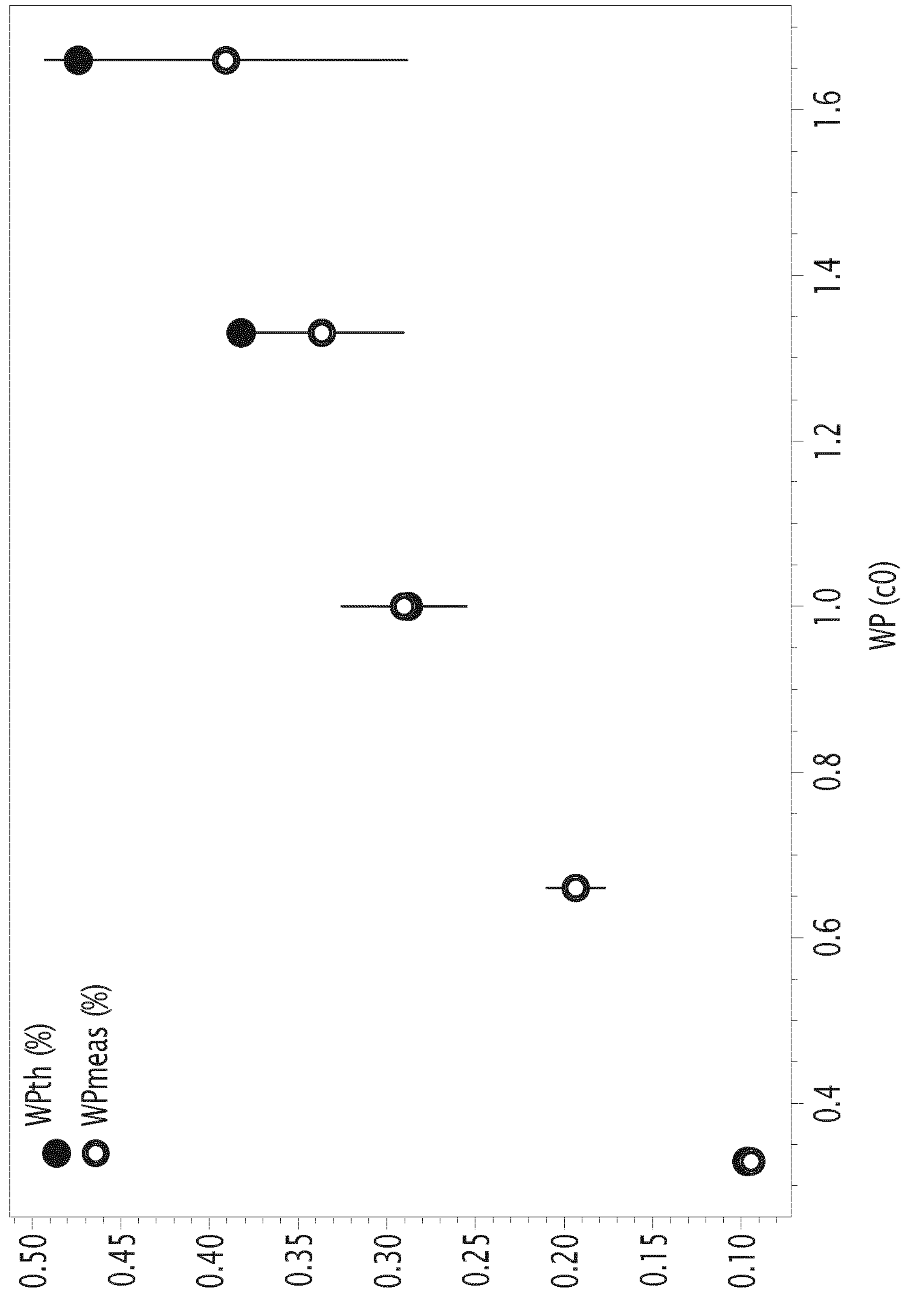
WPth (%)
WPmeas (%)
WP (c0)
WP (%)
0.4
0.6
0.8
1.0
1.2
1.4
1.6
0.50
0.45
0.40
0.35
0.30
0.25
0.20
0.15
0.10

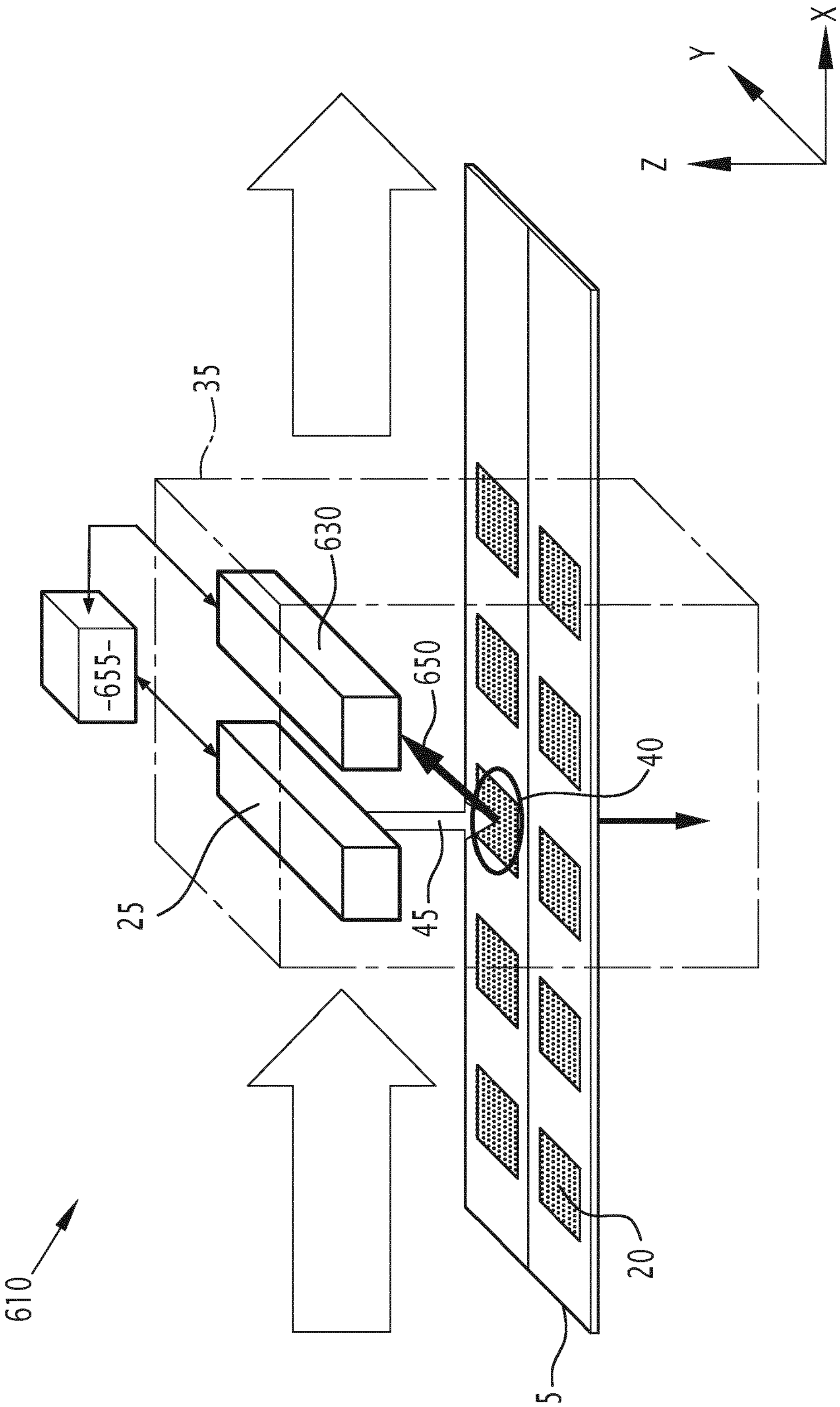


FIG.6

FIG.7

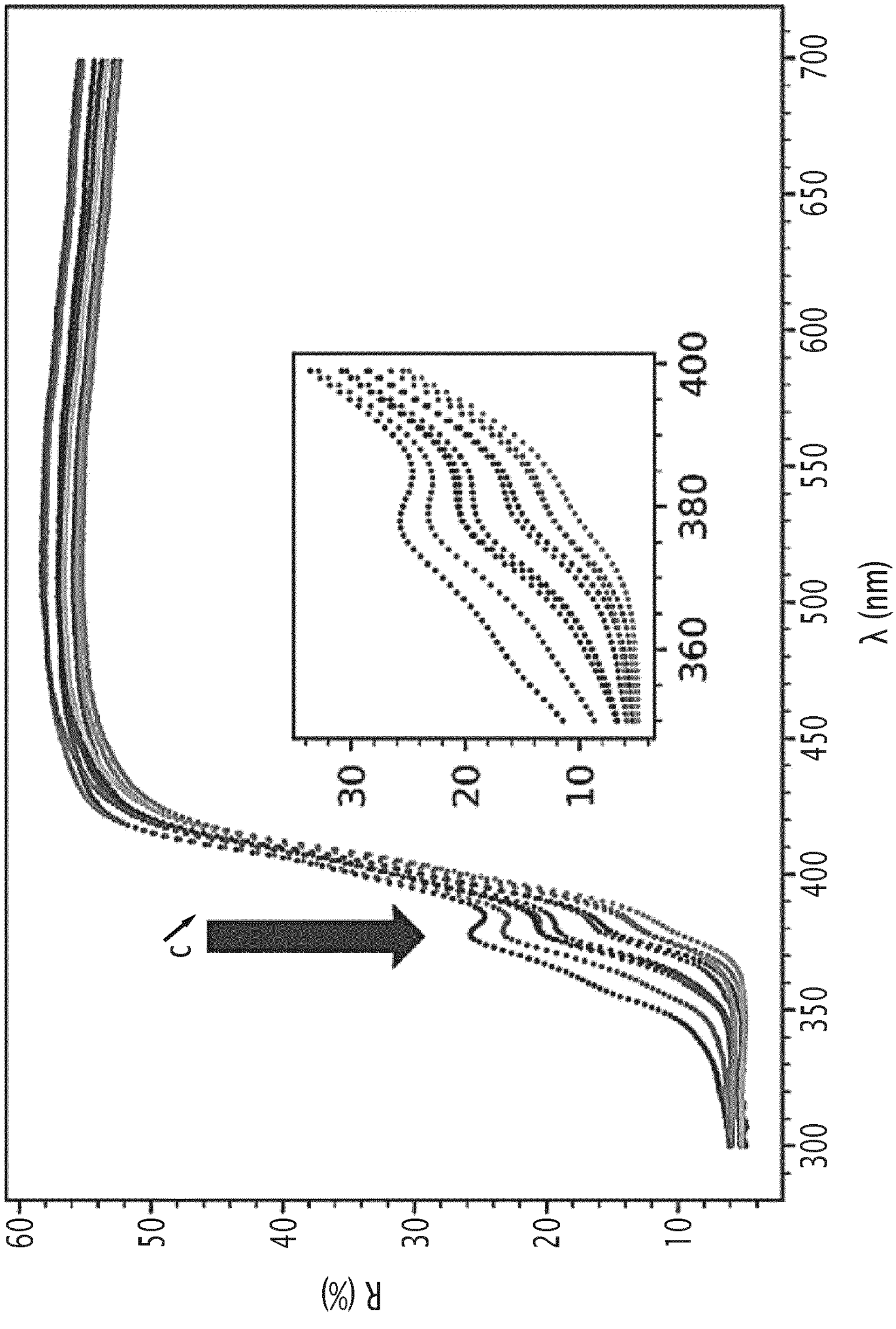
R (%)
60
50
40
30
20
10
λ (nm)
300
350
400
450
500
550
600
650
700
c
30
20
10
360
380
400

FIG.8

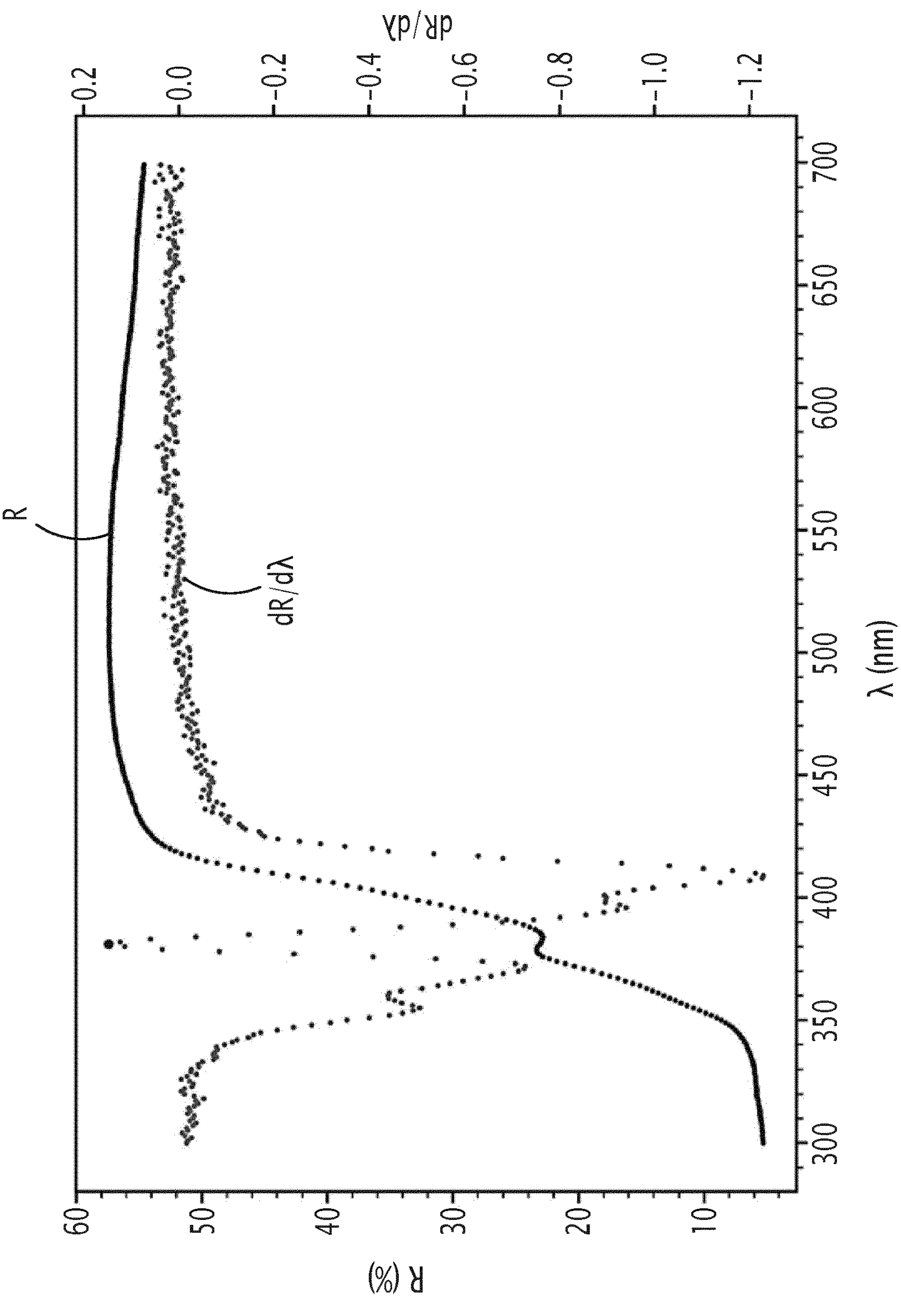
R (%)
dR/dλ
λ (nm)
R
dR/dλ
60
50
40
30
20
10
0.2
0.0
−0.2
−0.4
−0.6
−0.8
−1.0
−1.2
300
350
400
450
500
550
600
650
700

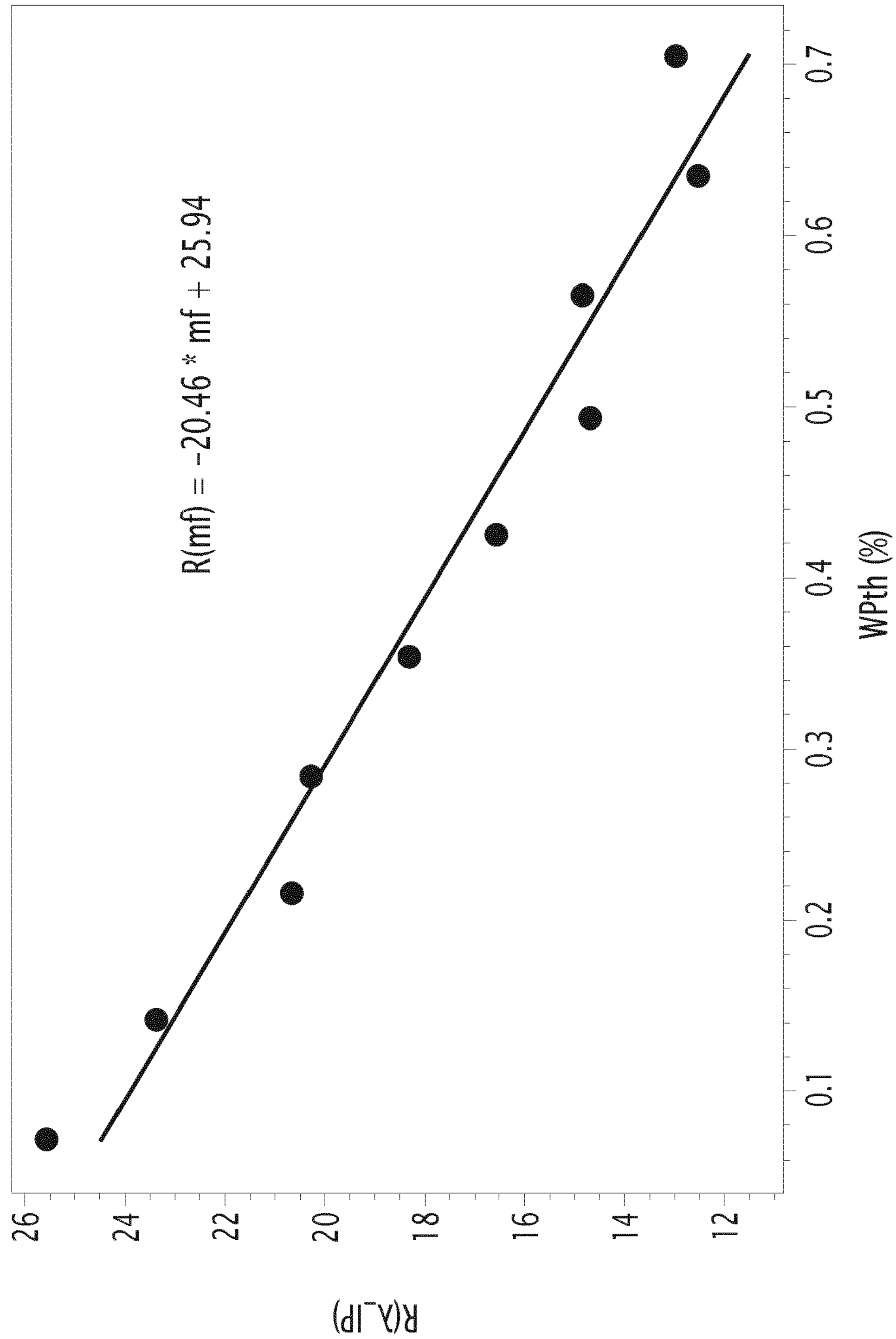
R(mf) = -20.46 * mf + 25.94
R(λ_IP)
26
24
22
20
18
16
14
12
0.1
0.2
0.3
0.4
0.5
0.6
0.7
WPth (%)

FIG.9

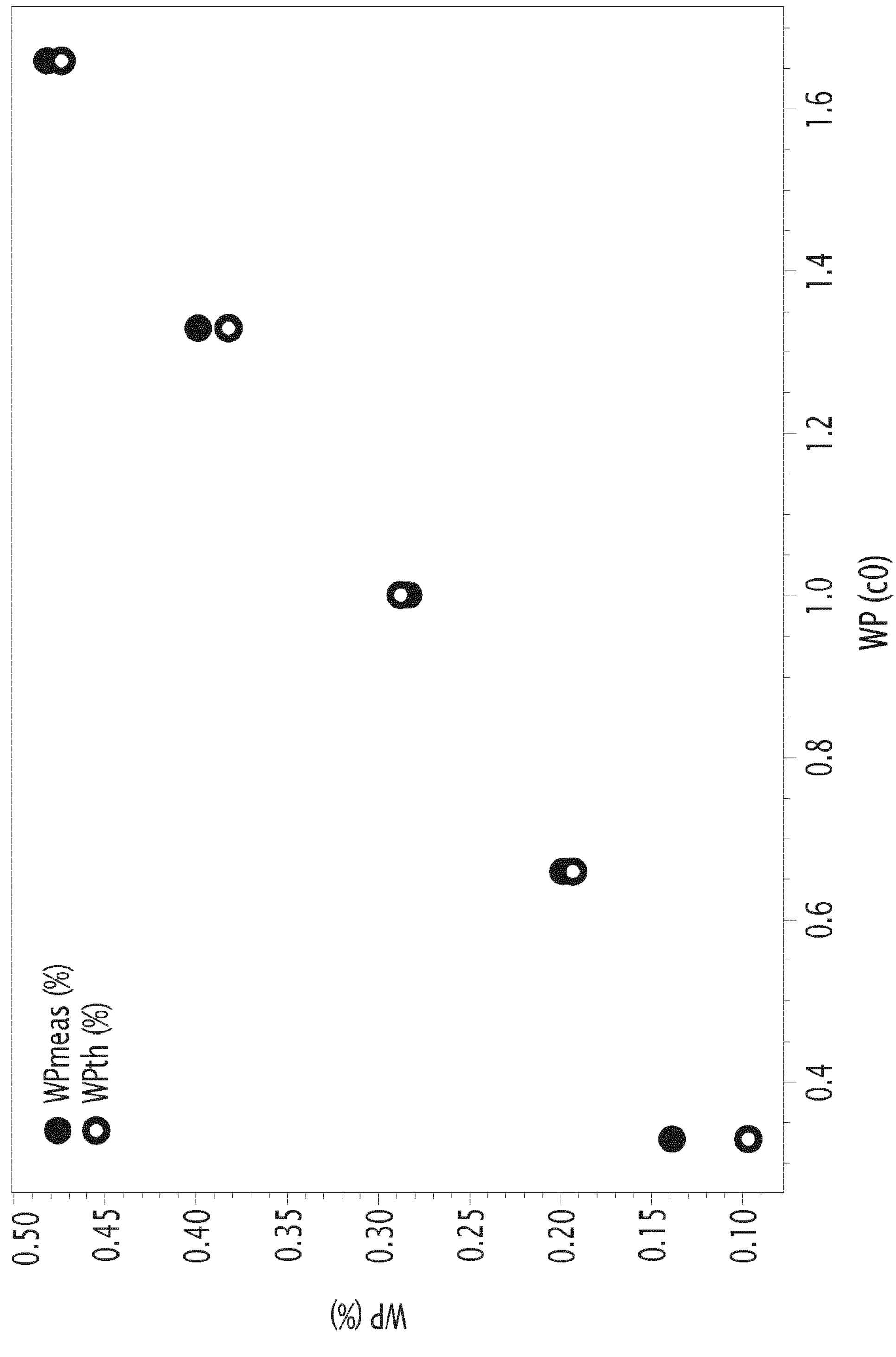
WPmeas (%)
WPth (%)
WP (%)
0.50
0.45
0.40
0.35
0.30
0.25
0.20
0.15
0.10
0.4
0.6
0.8
1.0
1.2
1.4
1.6
WP (c0)

FIG.10

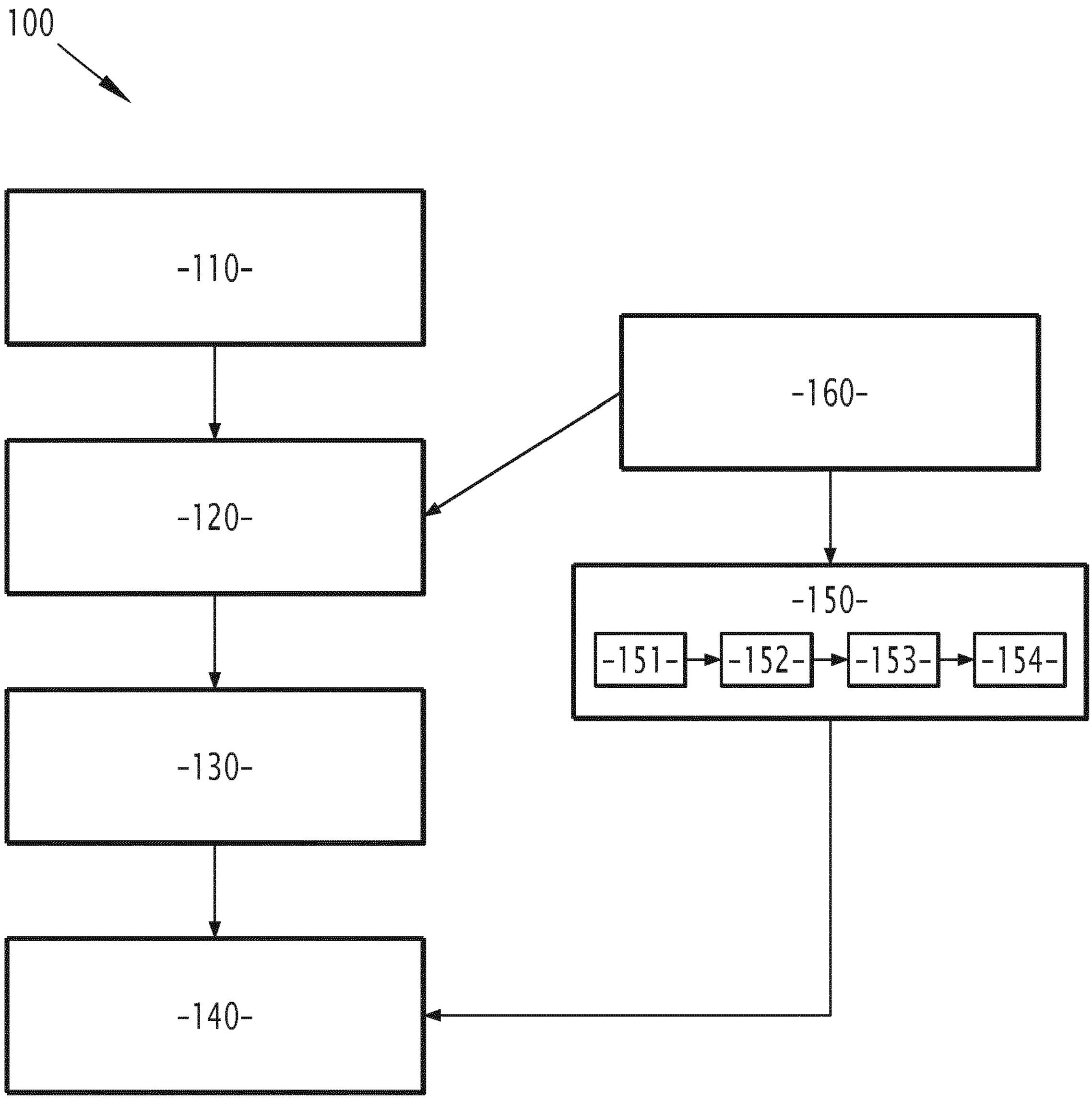


FIG.11

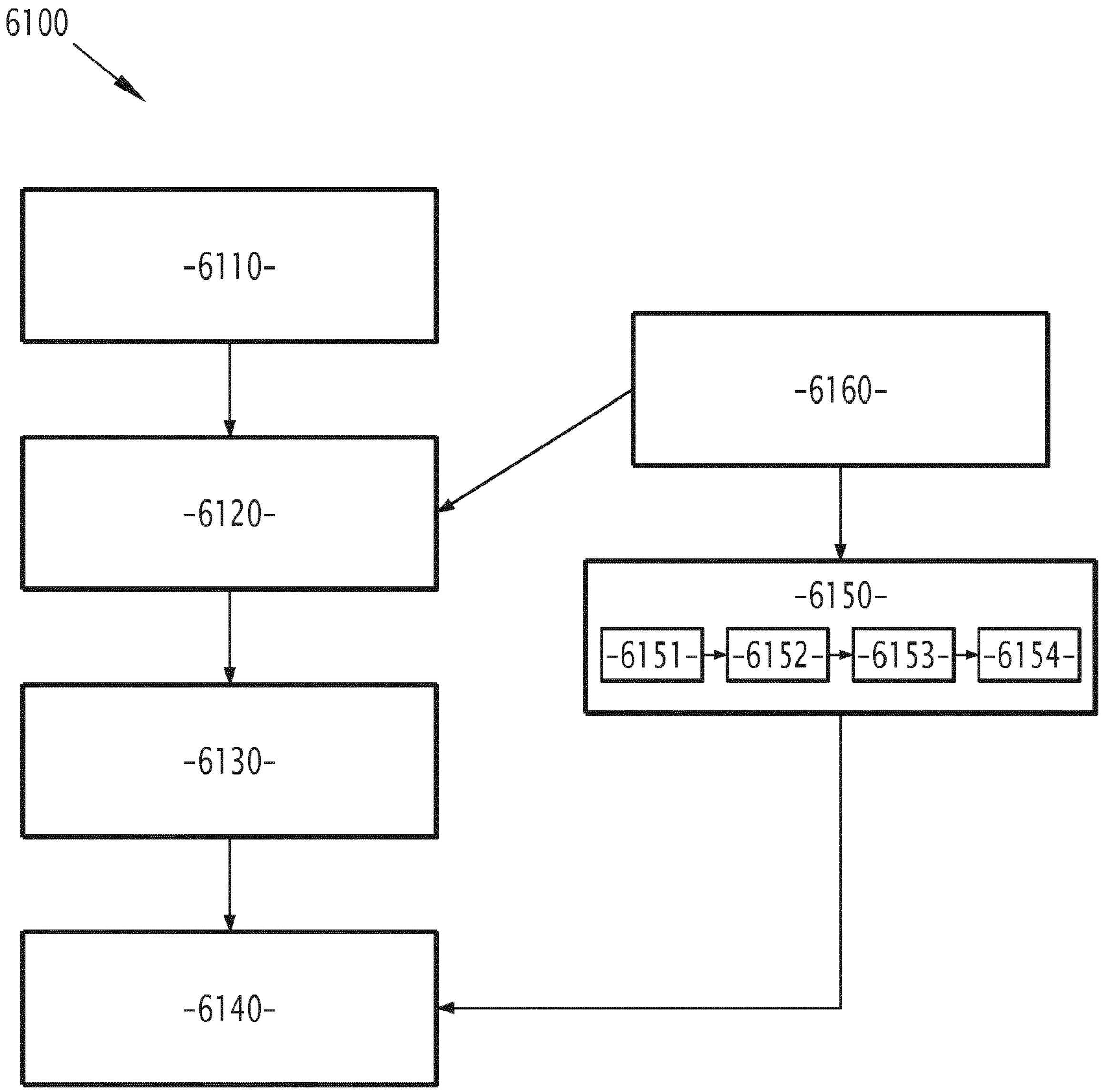


FIG.12

FIG.13

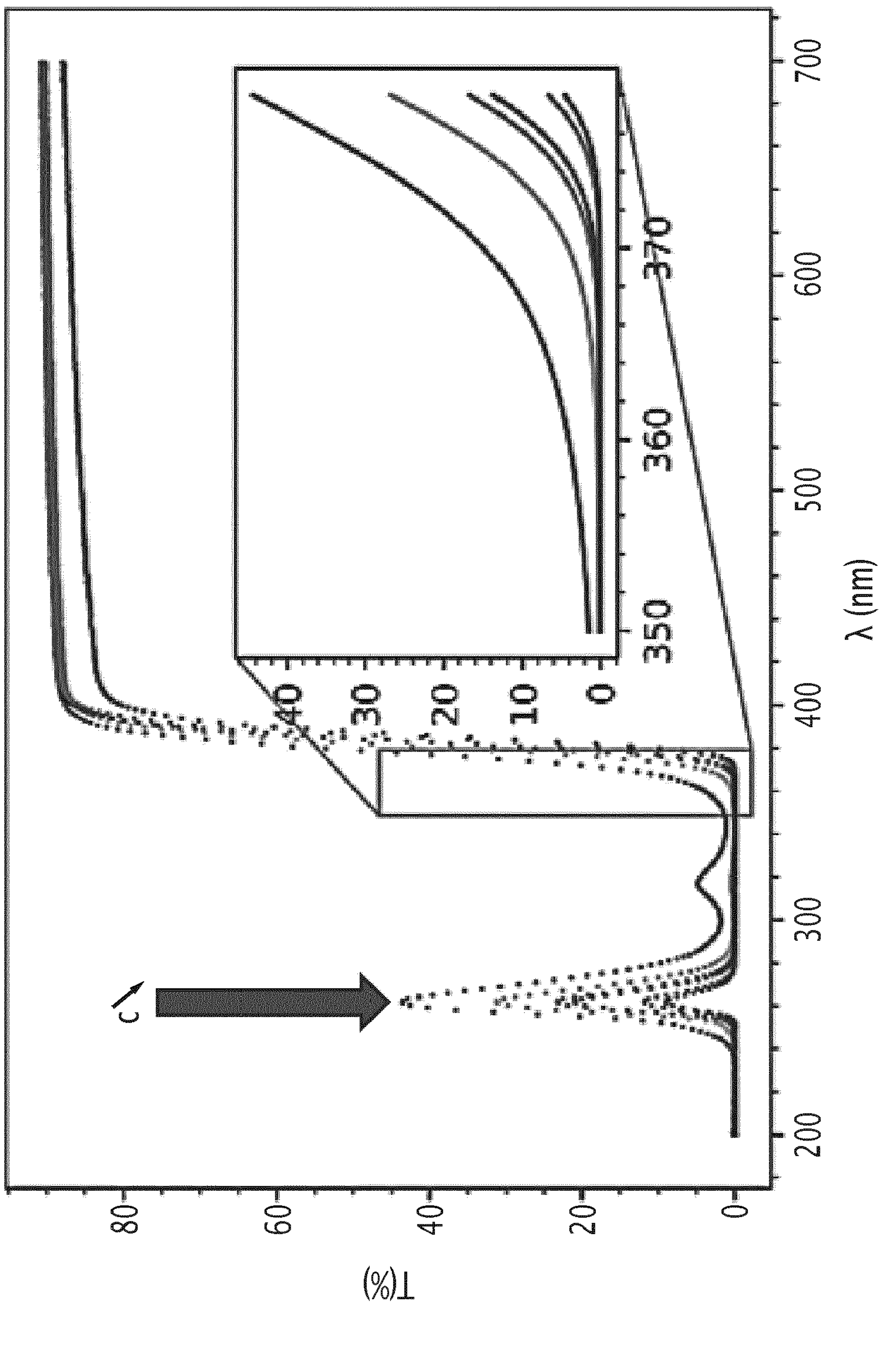
T(%)
0
20
40
60
80
200
300
400
500
600
700
λ (nm)
c
0
10
20
30
40
350
360
370

FIG.14

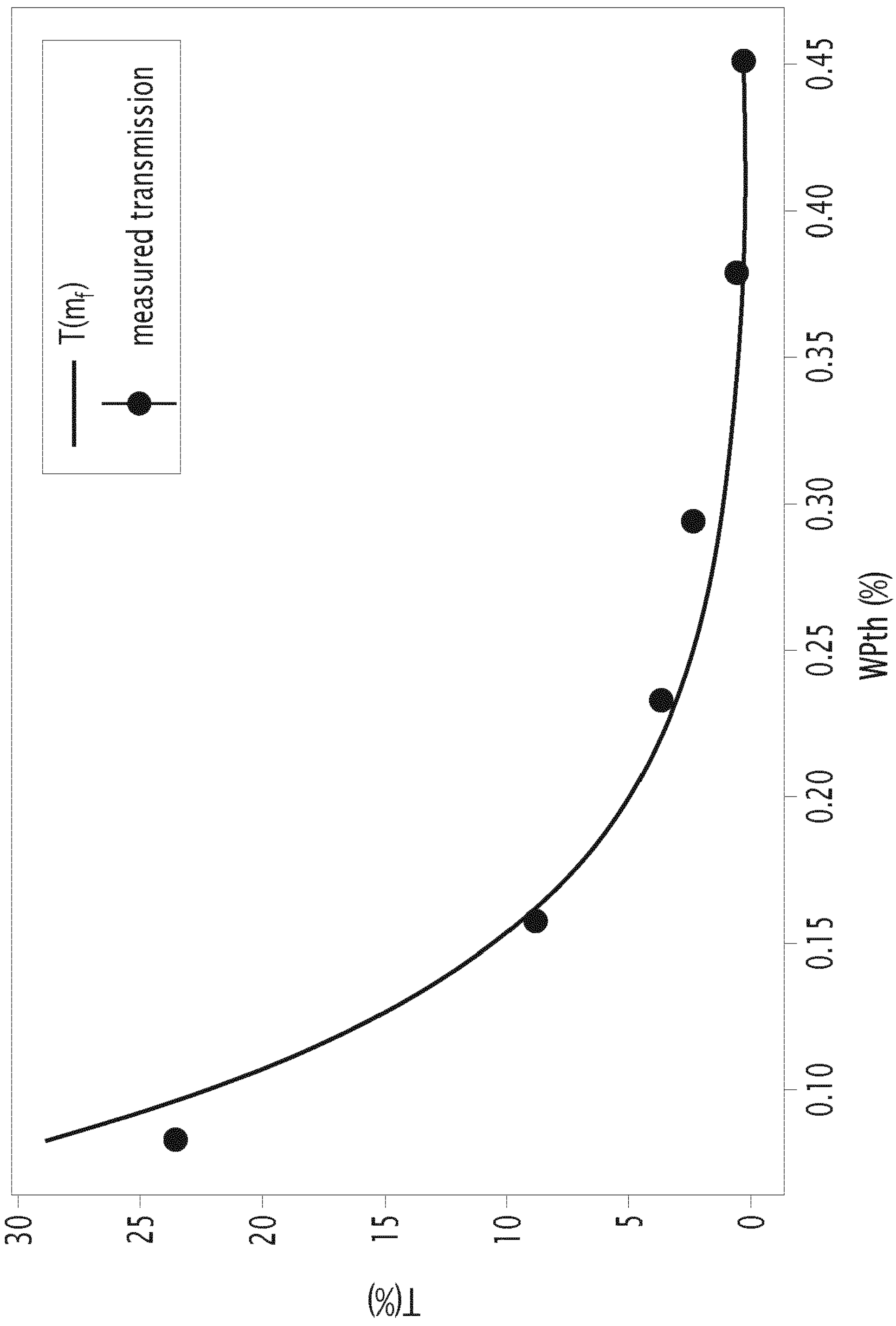
T(mf)
measured transmission
T(%)
WPth (%)
0
5
10
15
20
25
30
0.10
0.15
0.20
0.25
0.30
0.35
0.40
0.45

FIG.15

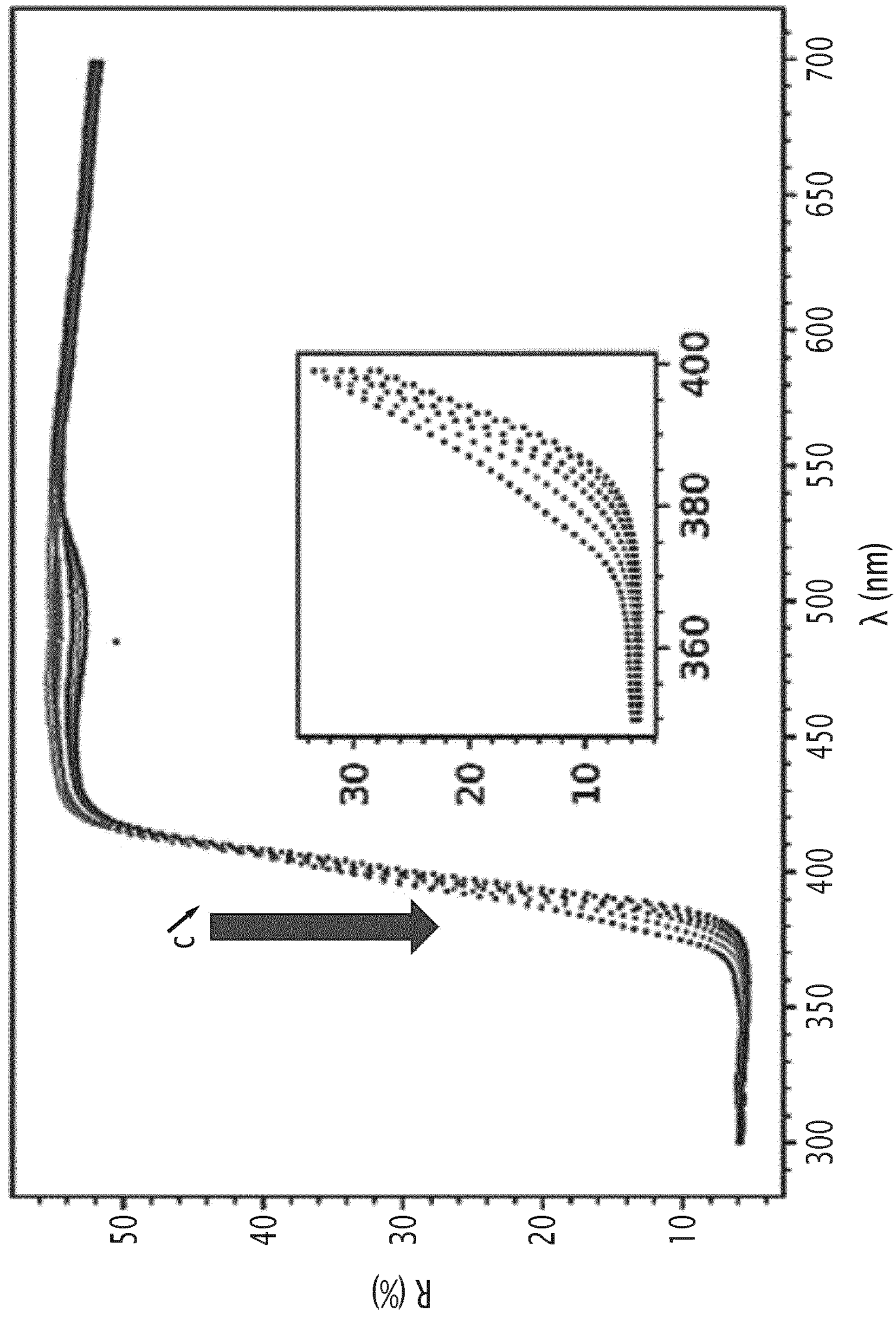
R (%)
λ (nm)
c
10
20
30
40
50
300
350
400
450
500
550
600
650
700
360
380
400

FIG.16

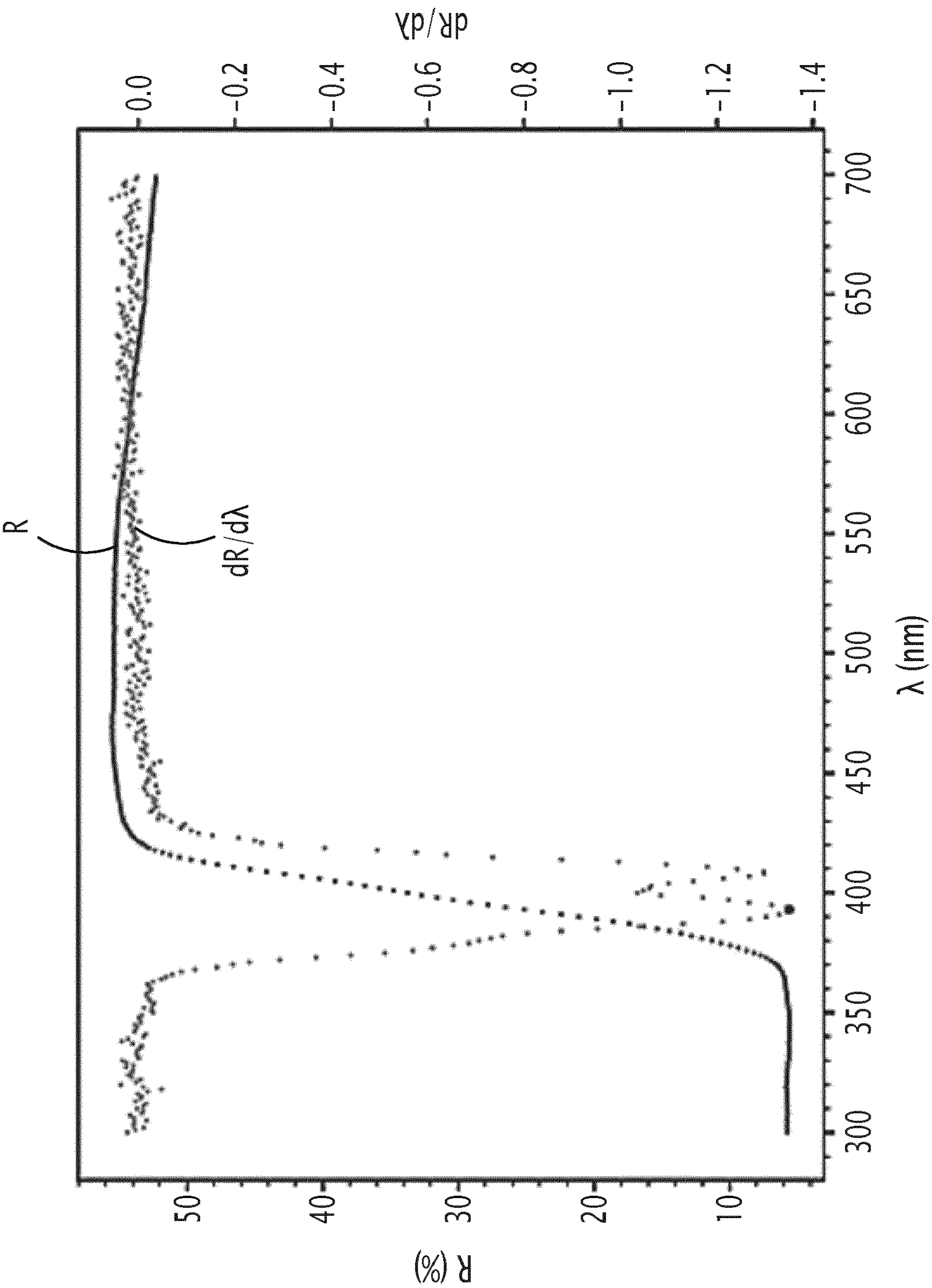

dR/dλ
0.0
-0.2
-0.4
-0.6
-0.8
-1.0
-1.2
-1.4
R
dR/dλ
R (%)
50
40
30
20
10
300
350
400
450
500
550
600
650
700
λ (nm)

FIG.17

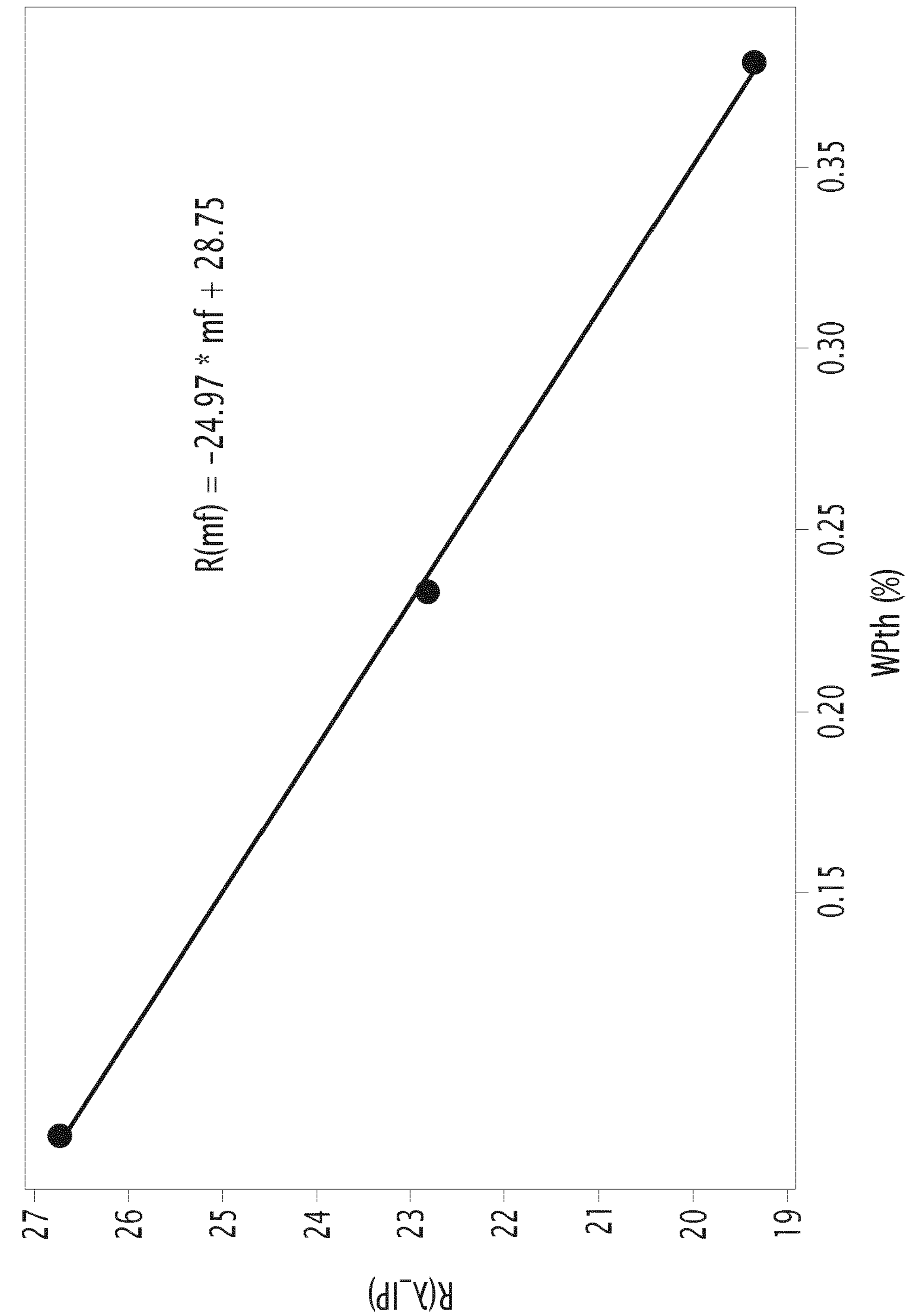
R(mf) = −24.97 * mf + 28.75
WPth (%)
R(λ_IP)
0.15
0.20
0.25
0.30
0.35
27
26
25
24
23
22
21
20
19

FIG.18

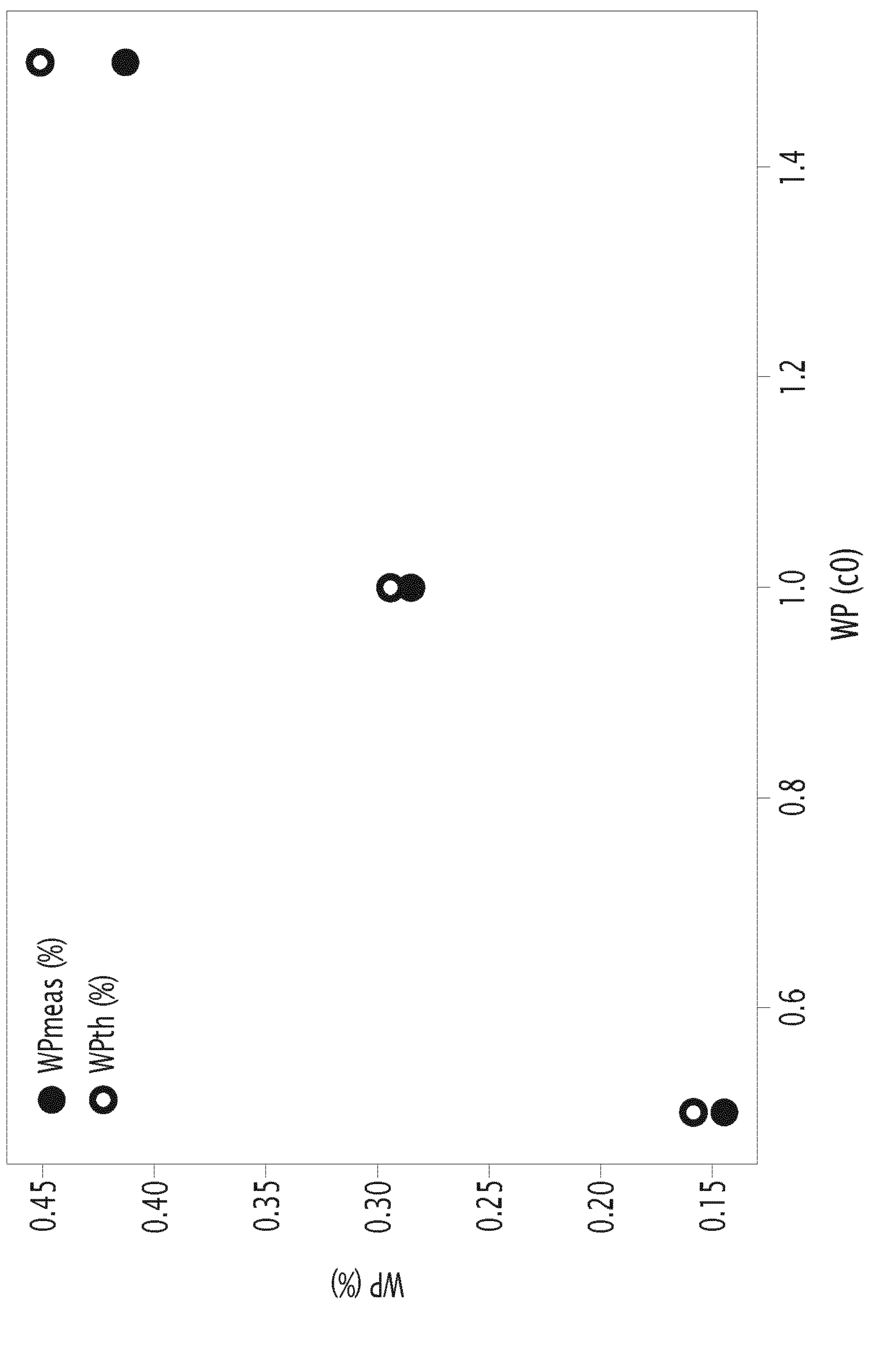

Europäisches Patentamt
European Patent Office
Office européen des brevets

# EUROPEAN SEARCH REPORT

Application Number
EP 24 30 5579

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CHO J W ET AL: "Ultraviolet reflective and mechanical properties of polyethylene mulching films", EUROPEAN POLYMER JOURNAL, PERGAMON PRESS LTD OXFORD, GB, vol. 37, no. 6, June 2001 (2001-06), pages 1227-1232, XP004230846, ISSN: 0014-3057, DOI: 10.1016/S0014-3057(00)00223-8 | 1-8, 12-15 | INV. G01N21/55 G01N21/86 G01N21/33 G01N33/442 G01N21/3563 |
| Y | * abstract; figures 3-4 * * Sections 2. and 3. * ----- | 3,4,9-12 | |
| X | CHU ZHU ET AL: "NEAR-INFRARED ANALYSIS OF CHEMICAL CONSTITUENTS AND PHYSICAL CHARACTERISTICS OF POLYMERS", APPLIED SPECTROSCOPY, THE SOCIETY FOR APPLIED SPECTROSCOPY. BALTIMORE, US, vol. 46, no. 1, January 1992 (1992-01), pages 69-72, XP000247306, ISSN: 0003-7028, DOI: 10.1366/0003702924444416 | 1-8, 12-15 | |
| Y | * the whole document * ----- | 9-11 | TECHNICAL FIELDS SEARCHED (IPC) |
| X | VIGERUST B ET AL: "QUANTITATIVE ANALYSIS OF ADDITIVES IN LOW-DENSITY POLYETHYLENE USING INFRARED SPECTROSCOPY AND MULTIVARIATE CALIBRATION", APPLIED SPECTROSCOPY, THE SOCIETY FOR APPLIED SPECTROSCOPY. BALTIMORE, US, vol. 45, no. 2, February 1991 (1991-02), pages 173-177, XP000175854, ISSN: 0003-7028, DOI: 10.1366/0003702914337605 | 1-8, 12-15 | G01N |
| Y | * the whole document * ----- | 9-11 | |

-/--

The present search report has been drawn up for all claims

1

| Place of search | Date of completion of the search | Examiner |
|---|---|---|
| Munich | 20 September 2024 | Riblet, Philippe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 24 30 5579

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | RADICE S ET AL: "Additive distribution in ethylene-chlorotrifluoroethylene alternating copolymer: A spectroscopic study", POLYMER DEGRADATION AND STABILITY, BARKING, GB, vol. 90, no. 2, November 2005 (2005-11), pages 390-394, XP027766609, ISSN: 0141-3910 [retrieved on 2005-11-01] | 1-8, 12-15 | |
| Y | * the whole document * | 9-11 | |
| | ----- | | |
| X | WIETZKE ET AL: "Determination of additive content in polymeric compounds with terahertz time-domain spectroscopy", POLYMER TESTING, ELSEVIER, AMSTERDAM, NL, vol. 26, no. 5, August 2007 (2007-08), pages 614-618, XP022133526, ISSN: 0142-9418, DOI: 10.1016/J.POLYMERTESTING.2007.03.002 | 1,2,5-8, 13-15 | |
| Y | * the whole document * | 9-11 | TECHNICAL FIELDS SEARCHED (IPC) |
| | ----- | | |
| Y | EP 3 778 759 A1 (ZHIJING NANOTECH CO LTD [CN]) 17 February 2021 (2021-02-17) * figure 11 * | 3,4,12 | |
| | ----- | | |
| Y,D | HEIDRICH ROBERT ET AL: "Quantification of UV protecting additives in ethylene-vinyl acetate copolymer encapsulants for photovoltaic modules with pyrolysis-gas chromatography-mass spectrometry", POLYMER TESTING, ELSEVIER, AMSTERDAM, NL, vol. 118, 27 December 2022 (2022-12-27), XP087242431, ISSN: 0142-9418, DOI: 10.1016/J.POLYMERTESTING.2022.107913 [retrieved on 2022-12-27] * Section 2. * | 9-11 | |
| | ----- | | |

The present search report has been drawn up for all claims

1

| Place of search | Date of completion of the search | Examiner |
|---|---|---|
| Munich | 20 September 2024 | Riblet, Philippe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.....................................................................................................
& : member of the same patent family, corresponding document

EPO FORM 1503 03.82 (P04C01)

page 2 of 2

# ANNEX TO THE EUROPEAN SEARCH REPORT ON EUROPEAN PATENT APPLICATION NO. EP 24 30 5579

This annex lists the patent family members relating to the patent documents cited in the above-mentioned European search report. The members are as contained in the European Patent Office EDP file on
The European Patent Office is in no way liable for these particulars which are merely given for the purpose of information.

20-09-2024

| Patent document cited in search report | Publication date | Patent family member(s) | Publication date |
| --- | --- | --- | --- |
| EP 3778759 A1 | 17-02-2021 | CN 110358237 A | 22-10-2019 |
| | | EP 3778759 A1 | 17-02-2021 |
| | | JP 7100391 B2 | 13-07-2022 |
| | | JP 2021517926 A | 29-07-2021 |
| | | KR 20210080280 A | 30-06-2021 |
| | | US 2021018662 A1 | 21-01-2021 |
| | | WO 2019196637 A1 | 17-10-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **HEIDRICH R. et al.** Quantification of UV protecting additives in ethylene-vinyl acetate copolymer encapsulants for photovoltaic modules with pyrolysis-gas chromatography-mass spectrometry. *Polymer Testing*, 2023, vol. 118, 107913
- *CHEMICAL ABSTRACTS*, 1843-05-6
- *CHEMICAL ABSTRACTS*, 52829-07-9
- *CHEMICAL ABSTRACTS*, 34443-12-4
- *CHEMICAL ABSTRACTS*, 1025-15-6
- *CHEMICAL ABSTRACTS*, 2530-85-0
- **ROBERT HEIDRICH et al.** UV lamp spectral effects on the aging behavior of encapsulants for photovoltaic modules. *Solar Energy Materials and Solar Cells*, 2024, vol. 266, 112674
- *CHEMICAL ABSTRACTS*, 3147-75-9